(19) Europäisches Patentamt
European Patent Office
Office européen des brevets

(11) Publication number: **0 571 087 A1**

# (12) EUROPEAN PATENT APPLICATION

(21) Application number: **93303123.9**

(22) Date of filing: **21.04.93**

(51) Int. Cl.[5]: **C07H 21/00**, C07H 19/10, C07H 23/00

A request for correction of the description has been filed pursuant to Rule 88 EPC. A decision on the request will be taken during the proceedings before the Examining Division (Guidelines for Examination in the EPO, A-V, 2.2).

(30) Priority: **18.05.92 US 885141**

(43) Date of publication of application: **24.11.93 Bulletin 93/47**

(84) Designated Contracting States: **AT BE CH DE DK ES FR GB GR IE IT LI LU MC NL PT SE**

(71) Applicant: **ROHM AND HAAS COMPANY**
**Independence Mall West**
**Philadelphia Pennsylvania 19105(US)**

(72) Inventor: **Bowers-Daines, Margaret Marie**
**107 North Stone Ridge Drive**
**Lansdale, Pennsylvania 19446(US)**
Inventor: **Miller, Muriel Elizabeth**
**509 East Montgomery Avenue**
**North Wales, Pennsylvania 19454(US)**
Inventor: **Holowach, Lorraine Pierce**
**611 Pennbrook Avenue**
**Lansdale, Pennsylvania 19446(US)**
Inventor: **Benkovic, Stephen James**
**1308 South Pugh Street**
**State College, Pennsylvania 16811(US)**

(74) Representative: **Harding, Charles Thomas et al**
**ROHM AND HAAS (UK) LTD.,**
**European Operations Patent Dept.,**
**Lennig House,**
**2 Mason's Avenue**
**Croydon CR9 3NB (GB)**

## (54) Cleavable phosphoramidites.

(57) A novel ribonucleoside phosphoramidite reagent for reversibly attaching functional groups to chemically synthesized polynucleotides is disclosed. In particular, this invention relates to a novel biotinylated reagent and method for its use in detection and purification of chemically synthesized polynucleotide sequences using avidin affinity chromatography. Chemical cleavage may be used to remove the functional group from the polynucleotide sequence to generate unmodified, biologically active DNA.

EP 0 571 087 A1

The present invention relates to cleavable phosphoramidites. In particular, the present invention relates to novel phosphoramidite reagents for attaching functional groups to chemically synthesized polynucleotides, and particularly to a novel biotinylated reagent that may be used for purification of full-length chemically synthesized polynucleotides using avidin affinity chromatography. Optionally, functional groups attached to polynucleotides using this novel reagent may be chemically cleaved from such polynucleotides, generating polynucleotides that are biologically active and suitable for *in vivo* use in experiments involving recombinant DNA.

The art prior art contains disclosures of three types of reagents for biotinylation of chemically synthesized polynucleotides: 1) activated phosphoramidite reagents to which the user adds biotin after the polynucleotide is cleaved from the solid synthesis support; 2) biotinylated phosphoramidites; and 3) a functionalized trityl reagent to which biotin may be attached after the polynucleotide is cleaved from the solid synthesis support.

Biotin may be attached to the 5' terminus of chemically synthesized, full-length polynucleotides. One class of reagents that may be used to biotinylate chemically synthesized oligonucleotides is composed of certain reagents that generate a primary amino, a sulfhydryl, or a hydroxyl group at the 5' terminus. A reagent molecule is added to full-length polynucleotide sequences as the last step of automated synthesis. Incomplete sequences, referred to in the art as failure sequences, and modified full-length polynucleotides are cleaved from the solid synthesis support, deprotected, and an excess of a biotin ester, such as biotin N-hydroxysuccinimide, is manually added to the crude synthesis mixture. Alter an incubation period, the excess biotin ester may be removed from the crude mixture, for example, by reverse phase, ion exchange or size exclusion chromatography. The oligonucleotides to which biotin has been coupled may be purified by avidin affinity chromatography.

In Coull, et al. (*Tetrahedron Lett*, 27:3991-2994 (1986)), it is disclosed that N-trifluoroacetyl-2-aminoethyl-(2-cyanoethyl)-N,N-diisopropylaminophosphorami dite may be attached to the 5' terminus of full-length DNA as the terminal step of automated synthesis, yielding full-length oligonucleotides having a primary amine at the 5' terminus.

Connolly (*Nucleic Acids Research*, 15:3131-3139 (1987)) discloses that N-monomethoxytrityl-O-methoxydiisopropylaminophosphinyl 3-aminopropan(1)ol may also be used to prepare chemically synthesized full-length polynucleotides having a primary amino group at their 5' termini.

Urdea, et al. (*Nucleic Acids Research*, 16(11) 4937-4956 (1988)) disclose the use of the deoxycytidine derivative $N^4$-[N-(6-trifluoroacetylamidocaproyl)-2-aminoethyl]-5'-O-dimethoxy-trityl-5-methyl-2'-deoxycytidine-3'-N,N-diisoprop ylmethylphosphora-midite to attach biotin esters to a free amino group at the 5' termini of full-length chemically synthesized polynucleotides.

U.S. 4,849,513 discloses nucleoside phosphoramidites having an aliphatic amino group attached to the sugar ring to attach biotin to the 5' terminus of chemically synthesized full-length polynucleotides.

U.S. 4,914,210 discloses linear polyether phosphoramidites having sulfhydryl, amino or hydroxyl moieties that may be used to attach one or more biotin molecules to the 5' terminus of full-length chemically synthesized polynucleotides.

Agrawal, et al. (*Nucleic Acids Research*, 14:6227-6245 (1986)) disclose a ribonucleoside derivative of uridine 5'-phosphoramidite to attach biotin to the 5' terminus of chemically synthesized full-length DNA. Agrawal, et al. teach the attachment of the phosphoramidite group at the 5' position of the ribose ring of uridine. Attachment of biotin is accomplished in several manual steps after the uridyloligonucleotide is cleaved from the solid synthesis support, by a first step to convert the ribose ring to a morpholine ring, followed by a second step in which biotin hydrazide is attached to the nitrogen of the morpholine ring. A disadvantage of this reagent is that the 5' morpholine-biotin derivative may not be chemically cleaved from the 5' terminus to generate unmodified, biologically active DNA.

Sproat, et al. (*Nucleic Acids Research*, 15:4837-4848 (1987)) disclose 5'-(S-triphenylmethyl) mercapto-2'-5'-dideoxyribonucleoside-3'-O-(2-cyanoethyl N,N-diisopropylphosphoramidites) have been used to prepare 5'-(S-triphenylmethyl) mercapto oligonucleotides. Addition of these reagents generates a free thiol moiety for manual attachment of biotin at the 5' terminus of full-length chemically synthesized polynucleotides.

Ansorge, et al. (*Nucleic Acids Research*, 15:4593-4602 (1987) and Ansorge, et al. (*J. Biochem Biophys Methods*, 13:315-324 (1986)) disclose 5'-(S-triphenylmethyl-3-mercaptopropyl) phospho linkers that may be used to prepare 5'-(S-triphenylmethyl) mercapto oligonucleotides. Addition of these reagents generates a free thiol moiety for manual attachment of biotin at the 5' terminus of full-length chemically synthesized polynucleotides.

Tang and Agrawal (*Nucleic Acids Research*, 18:6461 (1990)) disclose the use of non-nucleosidic phosphoramidite linker reagents to introduce amino alkyl groups to the 5' terminus of full-length chemically

synthesized polynucleotides. More than one biotin functional group may be attached at the 5' terminus to increase the sensitivity of detection of the biotinylated polynucleotide if it is used as a probe. Additionally, the biotin may also be used for avidin affinity purification of full-length polynucleotides.

Biotinylated phosphoramidites have been used to attach biotin to the 5' terminus of chemically synthesized polynucleotides. Biotinylated phosphoramidites are reagents to which the functional group biotin is covalently attached prior to automated DNA synthesis, and biotinylation of oligonucleotides is accomplished as a single addition cycle during automated synthesis.

Roget, et al. (*Nucleic Acids Research*, 17:7643-7651(1989)) disclose the use of 5'-O-(4,4'-dimethoxytrityl)-4-N-[6-N-biotinylaminohexyl]-2'-deoxycytidine-3'-O-(2-cyanoethyl)-N,N-diisopropylphosphoramidite to attach one or more biotin moieties directly to the 5' terminus of full-length chemically synthesized polynucleotides during automated synthesis. Biotin coupling during automated synthesis avoids further manual biotin addition steps after cleavage of the polynucleotides from the solid synthesis support. Addition of more than one biotin enhances the ability to detect the biotinylated sequence when it is used as an oligonucleotide probe. Additionally, attachment of biotin during automated synthesis avoids excess free biotin that may interfere with the purification of biotinylated full-length polynucleotides by avidin affinity chromatography. However, the 5'-O-(4,4'-dimethoxytrityl)-4-N-[6-N-biotinylaminohexyl]-2'deoxycytidine-3'-O-(2-cyanoethyl)-N,N-diisopropylphosphoramidite derivatives cannot be chemically cleaved from the 5' terminus to generate unmodified biologically active polynucleotides.

Pieles, et al. (*Nucleic Acids Research*, 18:4355-4360 (1990)) disclose the use of a modified 2'-deoxycytidine-3'-O-phosphoramidite carrying an N-t-butylbenzoyl-protected biotin on a long polar spacer arm to attach biotin directly to the 5' termini of full-length chemically synthesized polynucleotides as the terminal step of chemical synthesis.

EP 305201 (Protected Biotin Derivatives) discloses linear, biotinylated phosphoramidites to attach a single biotin moiety directly to the 5' terminus of chemically synthesized polynucleotides as the terminal step of automated synthesis.

Sproat, et al. (*Nucleic Acids Research*, 19:733-738 (1991)) disclose the use of 2'-deoxycytidine-3'-O-phosphoramidite carrying an N-t-butylbenzoyl-protected biotin to biotinylate 2'-O-allyl oligoribo-nucleotides, which are RNA sequences having 2'-O-allyl protecting groups on the 2' carbon of the ribose ring to stabilize the sequences against degradation by RNAses or the presence of base. 2'-Deoxycytidine-3'-O-phosphoramidite carrying an N-t-butylbenzoyl-protected biotin is added to the 5' terminus of the 2'-O-allyl ribonucleotide sequences. Although the authors do not discuss removal of 2'-O-allyl protecting groups, they may be removed by treatment with acid (Greene and Wuts, *Protective Groups in Organic Synthesis*, Second Edition, John Wiley & Sons, Inc., New York (1991)), making the ribonucleotide sequence susceptible to cleavage in the presence of base. However, the entire deprotected oligoribonucleotide would be cleavable after this treatment. This article does not disclose or suggest any recognition of the desirability to provide a molecule with differentially reactive protecting groups such that one or more specific 2'-O-allyl protecting groups may be selectively removed as a means to chemically cleave the biotin from the polyribonucleotide sequence to generate biologically active, unmodified polynucleotides.

Teoule, et al. (WO 90/08156 - Derivatives of Polyhydroxylated Molecules for Introducing at Least One Branching Site into an Oligonucleotide) disclose the use of polyhydroxylated phosphoramidites to attach one or more biotin moieties to the 5' terminus of chemically synthesized full-length polynucleotides. Teoule, et al. teach the use of branched, hydroxylated groups to introduce multiple biotin moieties onto a single oligonucleotide 5' terminus to enhance the detection of these biotinylated oligonucleotides when used as probes.

Ribonucleoside phosphoramidites are known, and polynucleotides consisting completely of ribonucleosides, or consisting of mixtures of ribonucleosides and deoxyribonucleosides may be made by automated synthesis. The presence of one or more cleavage oxygens in the polynucleotide sequence would confer no advantage in purification of chemically synthesized polynucleotides if no functional group were present at a position 5' to the cleavage oxygen on the polynucleotide sequence. Furthermore, when more than one ribonucleoside base is present in an RNA or RNA/DNA sequence, the site of chemical cleavage should be limited to a desired specific location, and the sequence should be inert to chemical cleavage until such time as the user intends cleavage to occur.

Gildea, et al. (*Tetrahedron Letters*, 31:7095-7098 (1990)) disclose the use of 4,4'-dimethoxytrityl, an acid-labile group routinely used for protecting 5' hydroxyl groups of chemically synthesized polynucleotides, to reversibly attach biotin to the 5' termini of full-length, chemically synthesized polynucleotides. Due to the acid-labile nature of the bond between the dimethoxytrityl group and the 5' hydroxyl moiety at the 5' terminus, the biotin moiety may be cleaved from the 5' terminus to yield unmodified, biologically active polynucleotides. However, addition of the biotin moiety to the 5' terminus requires two

separate manual steps following cleavage of the crude synthesis mixture from the solid support. Because the trityl reagent remains attached to the 5' hydroxyl of the 5' terminus of the polynucleotide, only one biotin moiety may be added at the 5' terminus, and the position of the biotin within the polynucleotide sequence is limited to the 5' terminus. Disadvantages of using this reagent for biotin attachment to polynucleotides include the instability of the dimethoxytrityl bond to the 5' hydroxyl group, extra manual processing steps required after automated synthesis to attach biotin to the 5' terminus, and the presence of free biotin to compete with biotinylated polynucleotide for binding sites on avidin affinity purification media.

Forster, et al. (*Nucleic Acids Research*, 13:745-761 (1985)) disclose the use of a photoactivatable analog of biotin, N-(4-azido-2-nitro-phenyl)-N'-(N-d-biotinyl-3-aminopropyl)-N'-methyl-1,3-propane-diamine (PAB, photobiotin) to attach biotin to single-stranded polynucleotides. However, the sites of biotin attachment are nonspecific. Furthermore, the number of biotins attached and their locations within the polynucleotide sequence cannot be controlled. Therefore, this reagent is not suitable for biotinylation of full-length polynucleotides and purification by avidin affinity chromatography.

Shimkus, et al. (*Proc. Nat. Acad. Sci.*, 82:2593-2597 (1985)) disclose the use of biotinylated nucleotide analogs that contain a cleavable disulfide bond joining biotin to purine or pyrimidine bases to purify biotinylated double-stranded polynucleotides by avidin affinity chromatography. However, such reagents must be enzymatically incorporated into double-stranded DNA and cannot be used for automated synthesis. Therefore, such reagents may not be used to purify full-length, chemically synthesized polynucleotides.

Polynucleotides containing a functional group may be bound to an affinity ligand fixed to a solid support for further enzymatic or chemical modification as taught by Bengstrom, et al. (*Nucleosides and Nucleotides*, 10:507-509(1991). Bengstrom, et al. teach the attachment of single-stranded polynucleotides having a noncleavable biotin at the 5' terminus to a solid avidin affinity support. The polynucleotide may be further modified, for example, second strand synthesis and restriction enzyme digestion may be performed while the polynucleotide is immobilized. An advantage of fixing the polynucleotide to a solid support is that changes of reaction conditions and enzymes are made by washing away unwanted reagents and replacing them with new reagents. No solvent extraction or precipitation steps are necessary. After all modifications are made, Bengstrom, et al teach the physical separation of the second strand from the biotinylated template by denaturation. A disadvantage of this method as taught by Bengstrom, et al. is that after the desired modifications are made to the original template, the original biotinylated template strand is left attached to the affinity support. Furthermore, the biotin moiety is irreversibly attached to the 5' terminus of the original template strand. Bengstrom, et al. do not teach the reversible attachment of the functional group to the polynucleotide sequence so that the original template sequence may also be detached from the affinity support.

Potera (*Genetic Engineering News*, June 1991, Mary Ann Liebert Inc., Publishers, New York) discloses the assembly of genes or gene fragments using chemically synthesized polynucleotides fixed to a solid synthesis support. Potera discloses the use of a chemically synthesized polynucleotide that is biotinylated at the 5' terminus. Potera teaches the addition of noncleavable functional groups to polynucleotide sequences, and the placement of a restriction enzyme digestion site near the 5' terminus to remove the functional group from the 5' terminus after the sequences are made double-stranded and optionally further modified. Introducing such restriction site as taught by Potera is laborious and may require introducing undesirable or unacceptable changes in the polynucleotide sequence to create a suitable enzyme recognition sequence. Potera does not teach the placement of a chemically cleavable site within the polynucleotide phosphodiester backbone to remove the functional group in a manner that is independent of the nucleotide sequence at the site of cleavage.

It is an object of this invention to provide a reagent for reversible attachment of functional groups to chemically synthesized polynucleotides. Attachment of functional groups permits the purification or detection of sequences to which the functional group is attached. At a desired time, the functional group may be chemically cleaved from the sequence to generate unmodified, biologically active polynucleotides.

It is a further object of this invention to provide an improved process for preparing highly pure, full-length chemically synthesized polynucleotide sequences that are biologically active, using affinity chromatography.

It is yet a further object of this invention to provide a biotinylated ribonucleoside phosphoramidite reagent for reversible attachment of functional groups to chemically synthesized polynucleotides that may be attached in a single automated step, at any position within the polynucleotide sequence, during automated solid-phase synthesis.

According to a first aspect of the present invention there is provided a process comprising a step (a) comprising attaching a functionalized ribonucleoside at any position along a desired full-length chemically synthesized polynucleotide, such as a polydeoxyribonucleotide, wherein the ribonucleoside has a cleavable

oxygen, a ribose ring, a removable protecting group on the cleavage oxygen and a functional group appropriate for affinity separation attached to the ribose ring.

Preferably, the chemically synthesized polynucleotide has a 2'-oxygenated ribonucleotide bearing a first protecting group.

Preferably, the ribonucleoside has a second protecting group on its cleavage oxygen which is different than the first protecting group on the 2'-oxygen of the ribonucleotide and which can be removed under conditions which do not remove the first protecting group from the ribonucleotide in the polynucleotide sequence.

Preferably, the ribonucleoside is attached to the 5' terminus of the polynucleotide.

Preferably the polynucleotide contains a 2'-oxygenated ribonucleotide bearing the protecting group.

Preferably, the process comprises a further step (b) comprising binding the polynucleotide to a binding partner appropriate for the functional group on an affinity support.

Preferably, the process comprises a further step (c) comprising separating the bound functionalized polynucleotide from unlabeled failure sequences in order to yield purifed polynucleotide.

Preferably, the process comprises a further step (d) comprising removing the ribonucleoside from the purified polynucleotide.

Preferably, step (d) comprises subjecting the purified polynucleotide to appropriate conditions for a period of time sufficient to cause the protecting group on the cleavage oxygen to be removed, and then subjecting the polynucleotide to basic conditions for a time sufficient to cause cleavage of the phosphodiester bond which links the ribonucleoside to the polydeoxyribonucleotide.

Preferably, the ribonucleoside is attached as a phosphoramidite in the D+1 step of the phosphoramidite chemical synthesis of a polynucleotide which contains D nucleotides.

Preferably, step (a) comprises adding from about 1 to about 6 ribonucleosides in series, at least one of said ribonucleosides bearing said functional group.

Preferably, the ribonucleoside is uridine.

Preferably, the functional group is biotin or 2-(4-hydroxyphenylazo)benzoic acid.

Preferably, the affinity support is monomeric avidin or monomeric streptavidin, preferably tetrameric avidin or tetrameric streptavidin, or an antisense polynucleotide.

According to a second aspect of the present invention there is provided a compound having the structure:

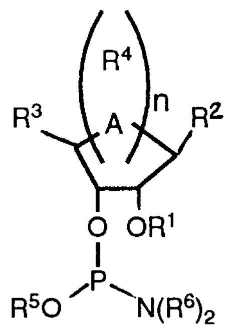

wherein A is oxygen, carbon, nitrogen, sulfur, or dimethylene and n is 0 or 1; **$R^1$** is trimethylsilyl, t-butyldimethylsilyl (t-$BuMe_2Si$), diphenylmethylsilyl, or 2'-O-[1-(2-fluorophenyl)-4-methoxypiperi-din-4-yl], O-allyl, or O-methyl, wherein t-butyldimethylsilyl (t-$BuMe_2Si$) is preferred; **$R^2$** and **$R^3$** may be the same or different and are selected from the group consisting of a functional group directly attached to the ring structure, a functional group attached to the ring structure through a spacer chain, a heterocylic base attached to the ring structure with a functional group attached to the heterocyclic base, and a heterocyclic base, a functional group, and a spacer chain, wherein the heterocyclic base is attached to the ring structure and the functional group is attached to the heterocyclic base by the spacer chain, with the proviso that only one of **$R^2$** and **$R^3$** may contain a heterocyclic base.

Preferably, the heterocyclic base present in **$R^2$** or **$R^3$** is selected from the group consisting of uracil, adenine, guanine, thymine, cytosine, and their derivatives.

Preferably, the spacer chain is present and is selected from the group consisting of $-(CH_2)_6$-NH, $(CH_2)_n$-W-, and $-[(CH_2)_y$-**V**-$(CH_2)_z]_p$-**W**-, wherein **W** is an atom which bears the functional group, and is selected from the group consisting of oxygen, nitrogen, sulfur, and an oxidized form of sulfur and **V** is selected from the group consisting of oxygen, sulfur, 1,2-phenyl, 1,3-phenyl, 1,4-phenyl, and **NQ**, wherein **Q** is hydrogen

or a lower alkyl containing 1 to 4 carbons.

Preferably, in place of the functional group there is a reactive group suitable for subsequent attachment of a functional group is present.

According to third aspect of the present invention there is provided a compound having the structure of either

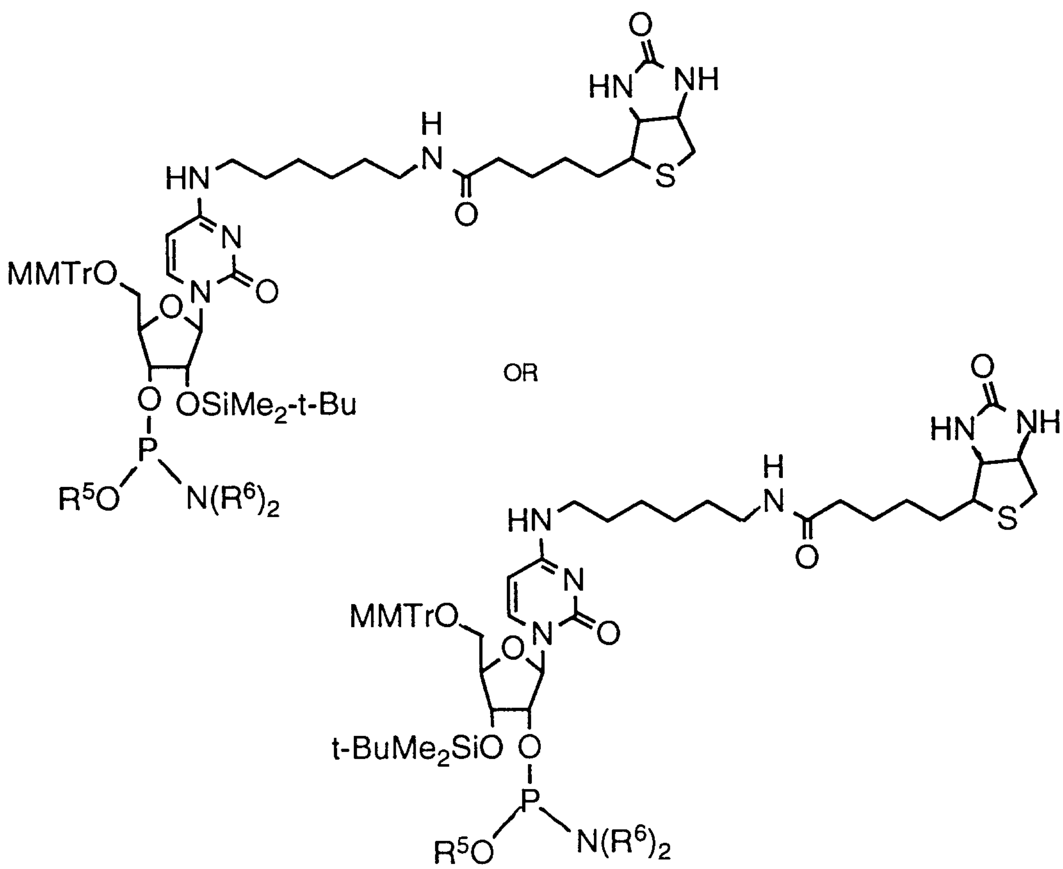

OR

wherein **R**$^5$ is beta-cyanoethyl, **R**$^6$ is isopropyl, MMTr represents methoxytrityloxymethyl and t-$BuMe_2Si$ represents t-butyldimethylsilyl.

The present invention therefore provides reagents that may be used to attach a functional group to a chemically synthesized polynucleotide sequence. The present invention also covers the aspect of a chemically synthesised polynucleotide sequence attached to an *in vivo* sequence.

In a preferred embodiment, the present invention provides a process for purifying chemically synthesized polydeoxyribonucleotides comprising: a) attaching a ribonucleoside to the 5' terminus of full length polydeoxyribonucleotide sequence products of chemical synthesis, and not to failure sequences, said ribonucleoside having a removable protecting group on its cleavage oxygen and a functional group appropriate for affinity separation attached to its ribose ring; b) contacting the chemically synthesized polynucleotide with an affinity support bearing a binding partner appropriate to the functional group on the ribonucleoside, to cause binding of the functionalized full-length sequence polydeoxyribonucleotides, and c) separating the bound functionalized full-length sequence polydeoxyribonucleotides from the unlabeled failure sequences. Preferably, the ribonucleoside is attached as a phosphoramidite in the D+1 step of the phosphoramidite chemical synthesis of a polydeoxyribonucleotide which contains D deoxyribonucleotides. Preferably, step (a) comprises adding from about 1 to about 6 ribonucleosides in series, at least one of said ribonucleosides bearing said functional group. Preferably, the process further comprises, after step (c), the step of removing the ribonucleoside from the purified polydeoxyribonucleotide by subjecting the functionalized polydeoxyribonucleotide to appropriate conditions for a time sufficient to cause the protecting group on the cleavage oxygen to be removed, and then subjecting the polynucleotide to basic conditions for a time sufficient to cause cleavage of the phosphodiester bond which links the ribonucleoside to the polydeoxyribonucleotide, yielding substantially pure unmodified full-length chemically synthesized polydeoxyribonucleotides.

In a preferred embodiment, the present invention provides a process for purifying chemically synthesized polynucleotides which contain 2'-oxygenated ribonucleotides bearing protecting groups comprising: a) attaching a ribonucleoside to the 5' termini of the full-length polynucleotide sequence products of chemical synthesis and not to the failure sequences, said ribonucleoside bearing a functional group appropriate for affinity separation attached to the ribose ring and having a removable protecting group on its 2' oxygen which is different than the protecting group on the ribonucleotides in the full-length sequence product, and can be removed under conditions which do not remove the protecting groups from the ribonucleotides in the full-length sequence product; b) contacting the chemically synthesized polynucleotide with an affinity support bearing a binding partner appropriate to the functional group on the ribonucleoside, to cause binding of the functionalized full-length sequence polydeoxyribonucleotides, and; c) separating the bound functionalized full-length sequence polydeoxyribonucleotides from the unlabeled failure sequences. Preferably, the ribonucleoside is attached as a phosphoramidite in the D+1 step of the phosphoramidite chemical synthesis of a polynucleotide which contains D nucleotides. Preferably, step (a) comprises adding from about one to about six 2'-oxygenated ribonucleosides in series, at least one of said ribonucleosides bearing said functional group. Preferably, the process comprises, as a step after step (c), the step of removing ribonucleoside and functional group from the purified polynucleotide by subjecting the functionalized polynucleotide to appropriate conditions for a time sufficient to cause the protecting group on the cleavage oxygen to be removed, while leaving the protecting groups on the 2'-oxygenated ribonucleotides in the full-length sequence in place, and then subjecting the polynucleotide to basic conditions for a time sufficient to cause cleavage of the phosphodiester bond which links the ribonucleoside of step (a) to the full-length sequence, yielding substantially pure unmodified full length chemically synthesized polynucleotides.

In a preferred embodiment, the present invention provides a process for reversibly functionalizing chemically synthesized polynucleotides which bear a first protecting group on 2'-oxygenated ribonucleotides of a desired sequence comprising: attaching a functionalized ribonucleoside at any position in a chemically synthesized polynucleotide sequence, said ribonucleoside bearing a second protecting group on its cleavage oxygen which is different than the first protecting group on the 2'-oxygen of the ribonucleotides in the polynucleotide sequence, and can be removed under conditions which do not remove the first protecting group from the ribonucleotides in the polynucleotide sequence, said ribonucleoside having a functional group attached to the ribose ring. Preferably, the ribonucleoside is attached as a phosphoramidite in the D+1 step of the phosphoramidite chemical synthesis of a polynucleotide which contains D nucleotides. Preferably, step (a) comprises adding from about one to about six 2'-oxygenated ribonucleosides in series, at least one of said ribonucleosides bearing said functional group. Preferably, the process comprises the steps of: (b) processing the functionalized polynucleotide in a manner which utilizes the functional group; and then (c) removing the ribonucleoside and functional group from the polynucleotide by subjecting the functionalized polynucleotide to appropriate conditions for a time sufficient to cause the protecting group on the cleavage oxygen to be removed, while leaving the protecting groups on the full length sequence in place and then subjecting the polynucleotide to basic conditions for a time sufficient to cause cleavage of the phosphodiester bond which links the ribonucleoside of step (a) to the full length sequence, yielding substantially pure unmodified full length chemically synthesized polynucleotides. Preferably, the processing comprises introducing the polynucleotide into a system and then detecting the location of the polynucleotide in the system through the characteristics of the functional group.

In a preferred embodiment, the present invention provides a process for reversibly functionalizing chemically synthesized polydeoxyribonucleotides comprising: a) attaching a functionalized ribonucleoside at any position in a chemically synthesized polydeoxyribonucleotide, said ribonucleoside having a removable protecting group on the cleavage oxygen and a functional group attached to the ribose ring. Preferably, the ribonucleoside is attached as a phosphoramidite in the D+1 step in a phosphoramidite chemical synthesis of a polydeoxyribonucleotide which contains D deoxyribonucleotides. Preferably, step (a) comprises adding from about 1 to about 6 ribonucleosides in sequence, at least one of said ribonucleosides bearing said functional group. Preferably, the process comprises the steps of: (b) processing the functionalized polydeoxyribonucleotide in a manner which employs the functional group; and then (c) removing the ribonucleoside and functional group from the polydeoxyribonucleotide by subjecting the functionalized polydeoxyribonucleotide to appropriate conditions for a time sufficient to cause the protecting group on the cleavage oxygen to be removed and then subjecting the polynucleotide to basic conditions for a time sufficient to cause cleavage of the phosphodiester bond which links the ribonucleoside to the polydeoxyribonucleotide, yielding unmodified chemically synthesized polydeoxyribonucleotides. Preferably, the processing comprises introducing the polynucleotide into a system and then detecting the location of the polynucleotide in the system through the characteristics of the functional group.

Preferably, the sugar of the ribonucleoside is ribose and the base is selected from the group consisting of adenine, guanine, cytosine, uracil and thymine. This aspect covers the situation of incorporating one or more RNA residues within a syntheitic sequence and using those for cleavage. In this regard, the RNA residue serves as a mechanism to cleave a functional group that is 5' to a desired DNA sequence. In this regard, the functional group has been incorporated directly onto the RNA molecule, but the functional group could also be one or more RNA or DNA residues removed from the cleavage RNA.

In a preferred embodiment, the reagents attach the functional group to a polynucleotide sequence in a manner in which the functional group may be cleaved from the polynucleotide sequence by chemical intramolecular hydrolysis of the phosphodiester bond that links the 5' terminus of the polynucleotide sequence to the functional group. An $\alpha$-hydroxy phosphodiester bond is cleaved through the interaction of a phosphorous atom with an oxygen atom on a carbon which is alpha to the carbon bearing the phosphodiester bond. Cleavage results in the formation of a five-membered cyclic phosphate by-product bearing the functional group and a polynucleotide having a hydroxyl group at carbon 5 of the ribose ring of the nucleotide base of the 5' terminus.

In another embodiment, the functional group may be cleaved from the polynucleotide sequence by chemical intramolecular hydrolysis of a $\beta$-hydroxy phosphodiester bond linking the 5' terminus of the polynucleotide sequence to the functional group through the interaction of a phosphorous atom with an oxygen atom on a carbon which is beta to the carbon bearing the phosphodiester bond. Cleavage results in the formation of a six-membered cyclic phosphate by-product bearing the functional group and a polynucleotide having a hydroxyl group at carbon 5 of the ribose ring of the nucleotide base of the 5' terminus.

The present invention therefore provides a method for reversible attachment of a functional group to the 5' terminus of full-length chemically synthesized polynucleotides, thus providing means to selectively retain desired sequences on an affinity support. The functional group may also be attached at any position within the polynucleotide sequence. Additionally, more than one functional group may be incorporated in series, or singly at more than one location within a polynucleotide sequence.

In a preferred embodiment, a biotinylated ***cis***- vicinal 2'-oxygenated phosphoramidite reagent may be used to attach one or more biotin moieties to full-length polynucleotide sequences when the reagent is added at the terminal step of automated synthesis. The resulting crude synthesis mixture of biotinylated, full-length sequences and nonbiotinylated, failure sequences may be passed in contact with avidin attached to a solid support, and the biotinylated polynucleotides will be selectively bound by the avidin. Nonbiotinylated failure sequences will not be bound by the avidin and may be washed away from the avidin attached to the solid support. Following removal of the failure sequences, highly pure, full-length sequences may be eluted away from the avidin. Optionally, at a desired time, the biotin moiety may be chemically cleaved from the polynucleotide sequence in a manner that generates a biologically active sequence.

The reagents and purification processes of the present invention provide an advantage over purification methods presently known in the art. Samples which would be difficult to purify by reverse phase chromatography or polyacrylamide gel electrophoresis due to low full-length sequence abundance, sequence length, or nucleotide base sequence composition can be purified using the reagents and processes of the invention.

Unless otherwise indicated, as used in the specification and claims, the following terms shall have the following meanings.

The term "polynucleotide" means a single-stranded or double-stranded nucleotide sequence of more than one base in sequence length. Single strands may be composed entirely of DNA, entirely of RNA, or may be a DNA/RNA hybrid sequence.

The term "functional group" means a molecule that imparts the chemical property or properties of the functional group to the polynucleotide to which it is attached, thereby permitting detection or isolation of that polynucleotide based upon the chemical properties of the functional group.

The term "functionalized reagent" means a molecule bearing a functional group that may be used to attach the functional group to a polynucleotide sequence.

The term "functionalized polynucleotide" means a sequence to which one or more functional groups are attached.

The terms "$\alpha$-hydroxy phosphodiester bond" and "$\beta$-hydroxy phospho-diester bond" mean phosphodiester bonds having a hydroxyl group appended to the respective carbon atoms in a position alpha to or beta to the carbon atom bearing the phosphodiester bond.

The term "cleavage oxygen" means the oxygen atom appended to the carbon which is $\alpha$- (in reagents which form a five-membered cyclic by-product) or $\beta$- (in reagents which form a six-membered cyclic by-product) to the carbon atom bearing the phosphorous of the phosphodiester bond to be cleaved. This oxygen establishes the carbon-oxygen-phosphorus linkage which forms the cyclic phosphate by-product of

chemical cleavage. The functional group remains attached to the cyclic phosphate by-product.

The term "protecting group" means a moiety appended to any second group, such as sulfhydryl, amino, or hydroxyl, so that the second group bearing the protecting moiety is not susceptible to chemical modification during automated synthesis, deprotection steps, or applications conditions while the protecting group is present. Examples of suitable protecting groups and their use may be found in Greene and Wuts, *Protective Groups in Organic Synthesis*, Second Edition, (John Wiley & Sons, Inc., New York (1991). Additionally, protecting groups generally used in nucleoside chemistry are described in *Oligonucleotide Synthesis: A Practical Approach*, (M.J. Gait, Ed., IRL Press, (1984)) and in a review by Kessel and Seliger (*Progress in the Chemistry of Organic Natural Products*, Vol. 32, pp 297-366, (1975)).

The present invention provides new reagents that may be used to reversibly attach functional groups to chemically synthesized polynucleotides. In a preferred embodiment, the reagents are used to attach one or more functional groups to a polynucleotide sequence so that at a desired time, the functional group may be cleaved from the polynucleotide sequence by chemical intramolecular hydrolysis of the phosphodiester bond linking the 5' terminus of the polynucleotide sequence to the functional group. In another embodiment, a functional group may also be located at one or more sites within the polynucleotide sequence. At each position where a reagent molecule is incorporated, a chemically cleavable phosphodiester bond is created, wherein a cleavage oxygen is located on a carbon alpha- or beta- to the carbon bearing the phosphorous of the phosphodiester bond to be cleaved. Reagents which have the cleavage oxygen on an $\alpha$-carbon is preferred. The chemically cleavable phosphodiester bond formed upon attachment of the reagent to the polynucleotide remains stable to chemical cleavage due to the presence of a protecting group on the cleavage oxygen until such time as chemical cleavage is desired. In a two-step cleavage procedure, the protecting group is first removed from the cleavage oxygen, then an hydrolysis reaction forms the polynucleotide sequence and a cyclic phosphate by-product bearing the functional group.

In a preferred embodiment, the functionalized ribonucleoside phosphoramidite reagent is attached to the 5' terminus of the desired polynucleotide, producing functionalized polynucleotide having a cleavage oxygen bearing a protecting group at the 2'- or 3'-position of the ribose ring derived from the reagent; the functional group is attached directly or indirectly to this same ribose ring. The functional group can then be used, for example, to separate the labeled polynucleotide sequence from unlabeled failure sequences. When the phosphodiester bond between this ribose ring and the remainder of the polynucleotide sequence is cleaved, the unmodified polynucleotide sequence is obtained.

In the compound (1) having the structure:

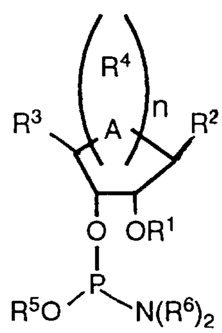

(1)

wherein **A** is oxygen, carbon, nitrogen, sulfur, or dimethylene and n is 0 or 1; $\mathbf{R^1}$ is trimethylsilyl, t-butyldimethylsilyl (t-BuMe$_2$Si), diphenylmethylsilyl, or 2'-O-[1-(2-fluorophenyl)-4-methoxypiperidin-4-yl], O-allyl, or O-methyl, wherein t-butyldimethylsilyl (t-BuMe$_2$Si) is preferred; $\mathbf{R^2}$ and $\mathbf{R^3}$ may be the same or different and are selected from the same group as each other consisting of a functional group directly attached to the ring structure, a functional group attached to the ring structure through a spacer chain, a heterocylic base attached to the ring structure and a functional group attached to the heterocyclic base, and a heterocyclic base, a functional group, and a spacer chain, wherein the heterocyclic base is attached to the ring structure and the functional group is attached to the heterocyclic base by the spacer chain with the proviso that only one of $\mathbf{R^2}$ and $\mathbf{R^3}$ may contain a heterocyclic base. When a heterocyclic base is present in $\mathbf{R^2}$ or $\mathbf{R^3}$, the heterocyclic base is selected from the group consisting of uracil, adenine, guanine, thymine and cytosine, their derivatives, and the like. Generally, any of the nucleoside purines or pyrimidine or their protected derivatives, or substituted purine or pyrimidine bases or their protected derivatives as disclosed in

United States Patent 4,849,513 may be used. Uracil is preferred because specific chemical modification for spacer chain attachment is facilitated through the reactive carbonyl moiety of uracil. When a spacer chain is present in **R²** or **R³**, the spacer chain is selected from the group consisting of - $(CH_2)_6$-NH, $(CH_2)_n$-**W**-, and -[$(CH_2)_y$-**V**-$(CH_2)_z$]$_p$-**W**-, wherein **W** is an atom which bears the functional group, and is selected from the group consisting of oxygen, nitrogen, sulfur, and an oxidized form of sulfur and **V** is selected from the group consisting of oxygen, sulfur, 1,2-phenyl, 1,3-phenyl, 1,4-phenyl, and **NQ**, wherein **Q** is hydrogen or a lower alkyl containing 1 to 4 carbons. In one embodiment, the spacer chain terminates in a reactive group, such as hydrogen attached to **W**, and the functional group may be added by reaction with the reactive group subsequent to automated synthesis. In the spacer chain, n is from 0 to 12, y is from 0 to 3, and z is from one to 3, and p is 1 or 2. In a preferred embodiment, the functional group on one of **R²** and **R³** is biotin, and the functional group on the remaining substituent is methoxytrityloxymethyl. Biotin is preferred because it may be used for purification of chemically synthesized polynucleotides using avidin affinity chromatography. Methoxytrityloxymethyl is preferred because it is less labile than dimethoxytrityloxymethyl. Methoxytrityloxymethyl may be also used for spectrophotometric quantitation of the polynucleotides to which oxygenated ribonucleoside phosphoramidite reagents are attached. The present invention also contemplates the use of other functional groups for **R²** or **R³**, including lipoic acid, 2-(4-hydroxyphenylazo)benzoic acid, trityloxy, dimethoxytrityloxymethyl, fluorscein, rhodamine, Texas Red, pyrene, digoxygenin, isoluminol, a paramagnetic spin label, electron-dense molecules such as ferritin and gold, fluorescent lanthanide chelates, enzymes such as horseradish peroxidase or alkaline phosphatase, 9-fluorenylethoxycarbonyl (Fmoc), or the like. Additionally, **R²** or **R³** may also be hydrogen, sulfur, nitrogen, hydroxyl or carboxymethyl. When **A** is carbon or dimethylene, **R⁴** is selected from the same group as **R²** and **R³**, consisting of hydrogen, sulfur, nitrogen, a functional group attached directly to the ring structure, and a functional group attached to the ring by a spacer chain. When **A** is dimethylene, then **R⁴** may be located on either carbon. When a spacer chain is present, the spacer chain is selected from the same group as for **R²** and **R³**. When a functional group is present on **R⁴**, the functional group is selected from the same group as **R²** or **R³**.

Additionally, a functional group may be added at **R²**, **R³**, or **R⁴** after the polynucleotide is cleaved from the solid synthesis support. For example, a reactive group such as hydroxyl, sulfhydryl, or amino may be located on the ribose ring, on the spacer chain, or on the heterocyclic base. In all cases, the reactive groups must be in protected form. The reactive groups are nucleophiles, and if not protected, could interfere with automated synthesis of the polynucleotide. The protecting group should be removable at a desired time, for example, during polynucleotide deprotection, so that an activated form of a functional group could be added subsequently. An example of an activated functional group is the N-hydroxysuccinimide ester of d-biotin. In a preferred embodiment, the reactive group would be placed on a spacer chain.

**R⁵** is selected from the group consisting of β-cyanoethyl, methyl, p-nitrophenethyl, o-chlorophenyl, and p-chlorophenyl, wherein β-cyanoethyl is generally preferred by those skilled in the art.

**R⁶** is selected from methyl, isopropyl or other lower alkyl, or **R⁶** may form a ring structure, as in a non-aromatic nitrogen-containing heterocycle, such as morpholino, piperidino, pyrrolidino or 2,2,6,6-tetramethylpiperidino, wherein isopropyl is preferred.

In the compound (2) having the structure:

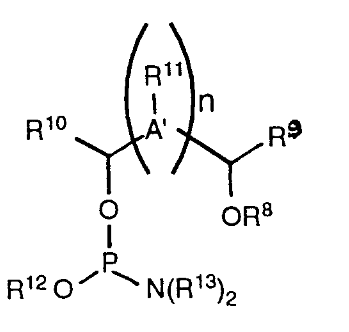

(2)

wherein **A'** is oxygen, carbon, nitrogen, sulfur, or dimethylene, and n is 0 or 1. **R⁸** is selected from the same group as **R¹**, wherein t-butyldimethylsilyl is preferred. When n is 1, **R⁹**, **R¹⁰** and when A' is carbon, nitrogen or dimethylene, **R¹¹** may be the same or different and are selected from the same group as **R²** and **R³**,

wherein inclusion of at least one biotin is preferred, as biotin may be used for purification of chemically synthesized polynucleotides using avidin affinity chromatography. The preferred composition of $\mathbf{R^{11}}$ is hydrogen. When **A'** is dimethylene, then $\mathbf{R^{11}}$ may be located on either carbon. Additionally, a functional group may be added at $\mathbf{R}^9$, $\mathbf{R}^{10}$, or $\mathbf{R^{11}}$ after the polynucleotide is cleaved from the solid polynucleotide synthesis support, in the same manner as for $\mathbf{R^2}$, $\mathbf{R^3}$, or $\mathbf{R}^4$. $\mathbf{R^{12}}$ is selected from the same group as $\mathbf{R}^5$. The preferred composition of $\mathbf{R^{12}}$ is $\beta$-cyanoethyl. $\mathbf{R^{13}}$ is selected from the same group as $\mathbf{R}^6$. The preferred composition of $\mathbf{R^{13}}$ is isopropyl.

$\alpha$-Hydroxy Phosphodiester Bond Cleavage

When chemically synthesized polynucleotides are functionalized using a biotinylated ribonucleoside phosphoramidite, (see Cleavage Reaction I), chemical cleavage generates a hydroxyl group at the 5 carbon position of the 5' terminal nucleotide base and a cyclic phosphate by-product bearing the functional group:

Cleavage Reaction I

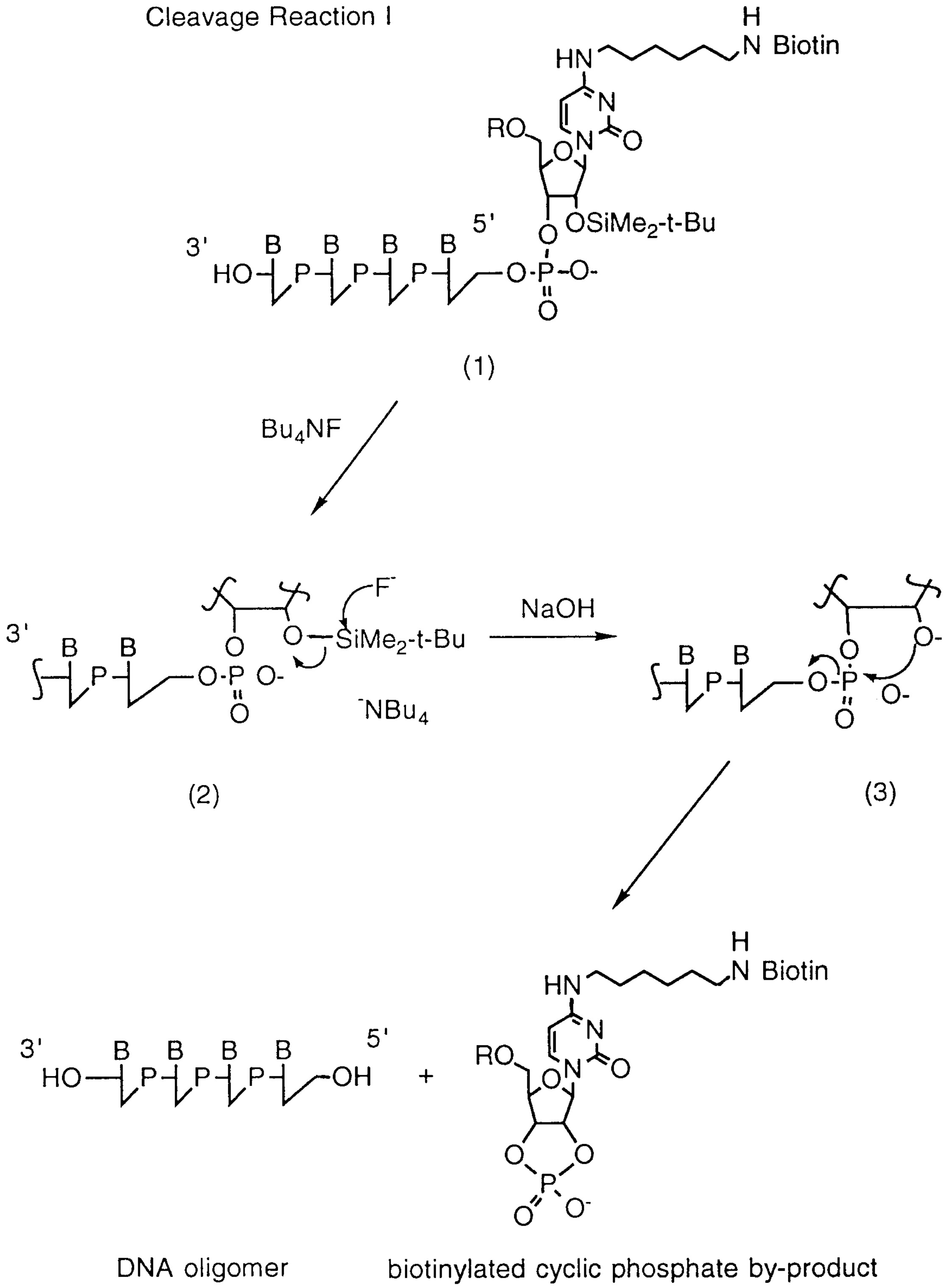


(1) (2) (3)

DNA oligomer (5)

biotinylated cyclic phosphate by-product (4)

wherein B is a heterocyclic base attached to the sugar-phosphate backbone of a polynucleotide sequence; R is hydrogen, methoxytrityl, or a polynucleotide sequence; $Bu_4NF$ is tetrabutylammonium fluoride. The numbers 3' and 5' refer to the 3' terminus and to the 5' terminus, respectively, of the chemically synthesized polynucleotide. Structure (1) in Cleavage Reaction I represents a polynucleotide bearing one biotin moiety at the 5' terminus. One biotinylated ribonucleoside phosphoramidite was attached as the

terminal step of automated synthesis. To initiate the cleavage reaction and remove biotin from the 5' terminus, the t-butyldimethylsilyl protecting group is removed from the cleavage oxygen, preferably by using tetrabutylammonium fluoride of about one molar in tetrahydrofuran (see (2) in Cleavage Reaction I). Following removal of the protecting group from the cleavage oxygen, alkoxide or hydroxide base of about one molar (see (3) in Cleavage Reaction I), for example, sodium hydroxide, may be used to cleave the α-hydroxy phosphodiester bond to form a five-membered cyclic phosphate by-product (see (4) in Cleavage Reaction I) bearing the functional group, and a biologically active polynucleotide sequence terminus (see (5) in Cleavage Reaction I).

More than one functionalized ribonucleoside phosphoramidite may be added in sequence:

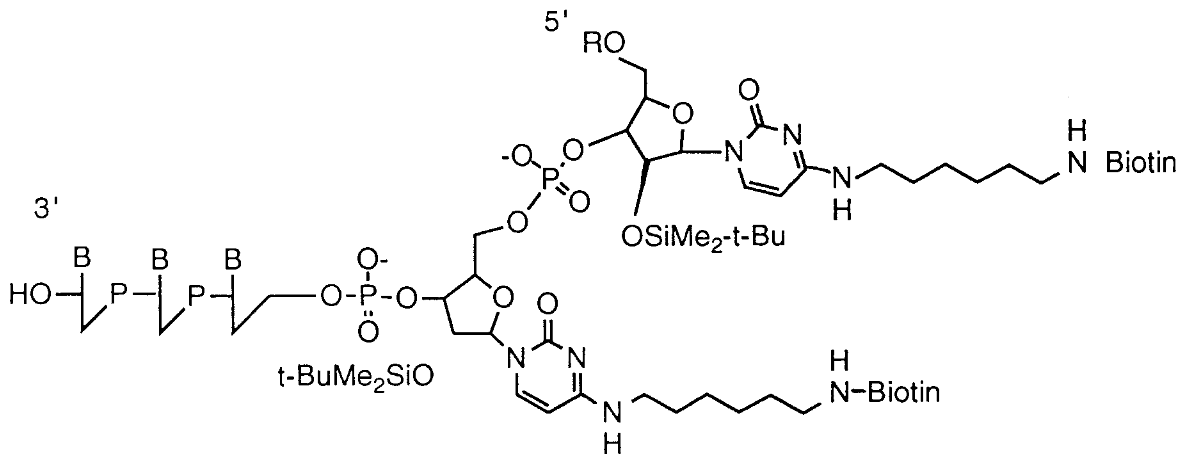

(6)

wherein in structure (6), B is a heterocyclic base attached to a sugar-phosphate backbone in a polynucleotide sequence; and R is hydrogen, methoxytrityl, or a polynucleotide sequence. The numbers 3' and 5' refer to the 3' terminus and to the 5' terminus, respectively, of the chemically synthesized polynucleotide.

If more than one α-hydroxy phosphodiester bond is incorporated into a polynucleotide sequence, each α-hydroxy bond may independently undergo cleavage by the cleavage reaction described above, and one cyclic phosphate may be generated for each α-hydroxy phosphodiester bond cleaved.

β-Hydroxy Phosphodiester Bond Cleavage

Chemical cleavage at the cleavage oxygen of a β-hydroxy phosphodiester bond generates an oligonucleotide sequence having a hydroxyl moiety at carbon 5 of the 5' terminus (see (11) of the Cleavage Reaction II below) and a six-membered cyclic phosphate by-product (see (10) of the Cleavage Reaction II below) bearing the functional group and as a result of according to Cleavage Reaction II described below. In Cleavage Reaction II, two functionalized phosphoramidite reagents have been attached in sequence to the 5' terminus of a chemically synthesized oligonucleotide. The structures illustrated in Cleavage Reaction II show the sequential cleavage of these reagent derivatives, and the cyclic phosphate by-product formed when each reagent derivative is cleaved.

Cleavage Reaction II

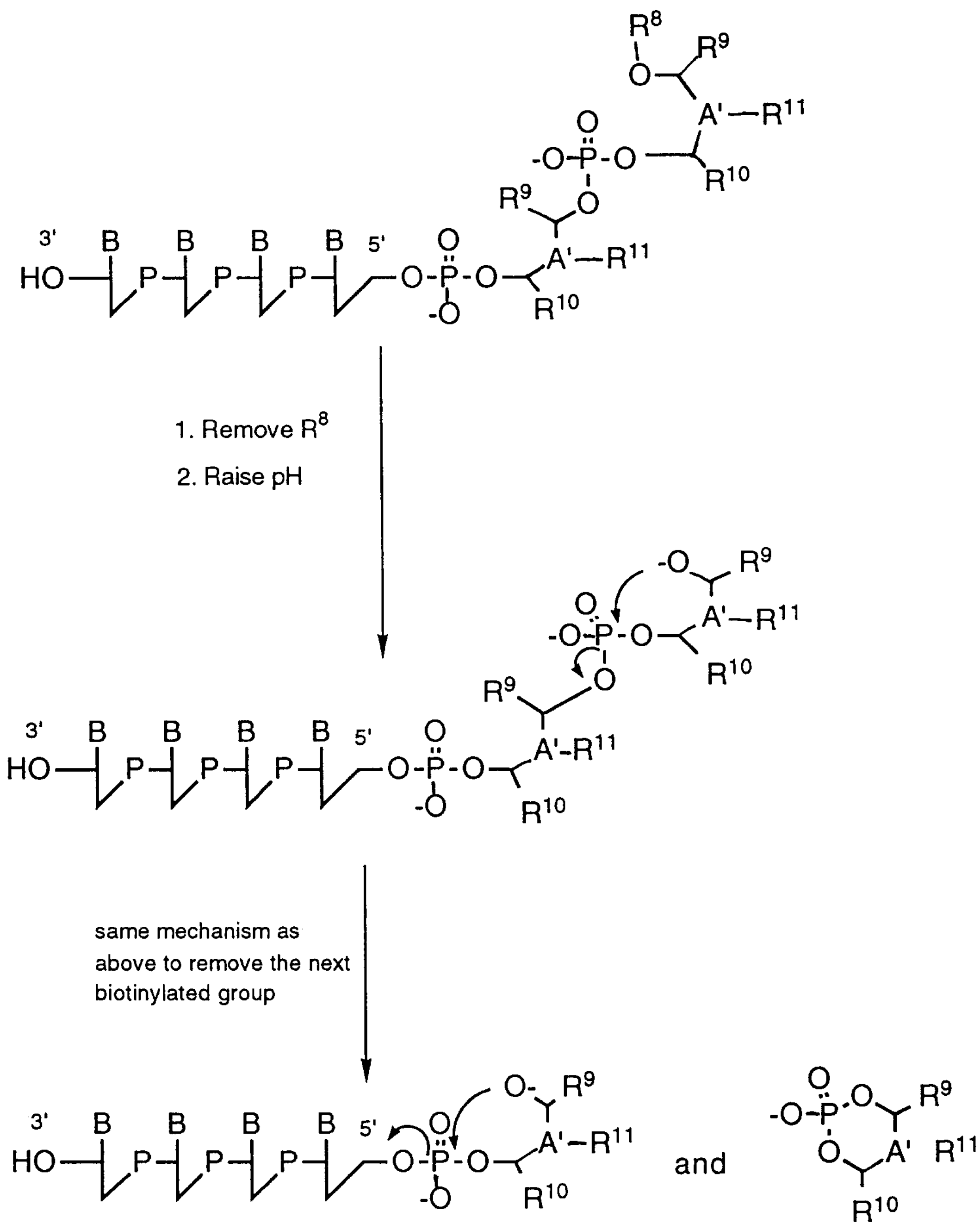


(7)

(8)

(9) and (10)

(Cleavage Reaction II, Cont'd)

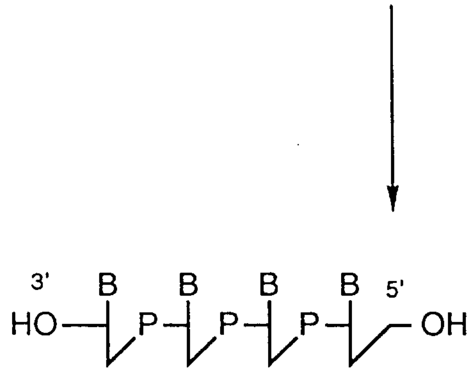

(11)

and

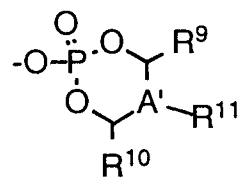

(10)

In Cleavage Reaction II, in structure (6), B is a heterocyclic base attached to a sugar-phosphate backbone in a polynucleotide sequence, and the numbers 3' and 5' refer to the 3' terminus and to the 5' terminus, respectively, of the chemically synthesized polynucleotide. In Cleavage Reaction II, $\mathbf{R}^8$ is labile, for example, monomethoxytrityl or dimethoxytrityl. $\mathbf{R}^8$ may not be nonlabile, for example, a polynucleotide sequence or a nucleoside base, as the oxygen protected by $\mathbf{R}^8$ could not be made available for cyclic phosphate formation.

DNA

In a preferred embodiment, a functional group is reversibly attached to chemically synthesized polydeoxyribonucleotides (synthetic DNA) using the functionalized, oxygenated ribonucleoside reagent of the present invention. A chemically cleavable $\alpha$-hydroxy phosphodiester bond may be created at a specific desired site in the DNA sequence, preferably at the 5' terminus. Attachment of the ribonucleoside reagent introduces into the DNA sequence a ribose moiety bearing a protected 2'- or 3'-oxygen atom to form an $\alpha$-hydroxy phosphodiester bond. When the protecting group is present on the 2'- or 3'-oxygen atom, the oxygen is not susceptible to cleavage under conditions encountered during automated synthesis, cleavage from the synthesis support, base deprotection, or applications generally used in the art.

Chemical cleavage of the functional group from the DNA sequence is accomplished as follows. DNA is stable to alkaline conditions because it is composed of 2'-deoxyribonucleotides and there are no 2'-hydroxyl groups on the ribose rings of DNA. However, 2'-oxygenated ribonucleotides are chemically cleavable when subjected to alkaline conditions, to form cyclic phosphate by-products (Lehninger, A., *Biochemistry*, 2nd Edition, Chapter 12, pp 322-323, Worth Publishers, Inc., New York, New York (1975)). When a functionalized oxygenated ribonucleotide phosphoramidite reagent is attached to a DNA sequence, a base-labile cleavage oxygen at the 2' position of the ribose ring of the functionalized reagent is introduced into the DNA sequence as an $\alpha$-hydroxyphosphodiester bond. The cleavage oxygen is made stable to base and also to conditions encountered during automated synthesis, cleavage from the synthesis support, base deprotection or in applications work by the attachment of a protecting group. Selective removal of the protecting group from the cleavage oxygen at a desired time renders the oxygen susceptible to cleavage under alkaline conditions, and thereby permits the removal of the functional group from the DNA sequence. A hydroxyl group is generated at carbon 5 of the 5' terminus of the DNA sequence.

In another embodiment, a functional group may be attached through a $\beta$-hydroxyphosphodiester bond, and subsequently cleaved in the presence of base by the Cleavage Reaction II.

RNA, RNA/DNA Hybrid Polynucleotides

Functional groups may also be reversibly attached to chemically synthesized polyribonucleotides (synthetic RNA) or DNA/RNA hybrid polynucleotides. In these molecules, $\alpha$-hydroxy phosphodiester bonds are normally found within the sugar-phosphate backbone at the 2'-position of the ribose ring of each RNA base.

In an embodiment of the present invention, functional groups may be reversibly attached at one or more specific sites within RNA or DNA/RNA hybrid polynucleotides to permit chemical cleavage of functional groups by Cleavage Reaction I or Cleavage Reaction II.

However, when RNA bases are present in a sequence, it is necessary to distinguish between the 2'-oxygen normally found on the ribose ring of RNA bases, and the cleavage oxygen of the α-hydroxy phosphodiester bond specifically intended for intramolecular phosphodiester bond cleavage.

Protecting Groups

For chemical oligonucleotide synthesis, a protecting group is normally attached to the 2'-oxygen of RNA bases during synthesis to protect the 2'-oxygen from undergoing hydrolysis during ammonia cleavage from the solid support and base deprotection. According to the present invention, when adding a chemically cleavable functional group to RNA or RNA/DNA hybrid molecules where a cleavage oxygen is present, a different protecting group is attached to the 2'-oxygens of the ribose rings than is attached to the cleavage oxygen so that selective deprotection of either the 2'-oxygens of the RNA bases or of the cleavage oxygen may be achieved. The protecting group on the cleavage oxygen should be selected so that deprotection renders only the α-hydroxy bond to be cleaved susceptible to hydrolysis. All other 2'-hydroxy bonds within the sequence should remain protected during the time that the polynucleotide is exposed to alkaline conditions until such time that cleavage is completed. In the present invention, the cleavage oxygen is protected from undergoing cleavage by attaching to the cleavage oxygen a protecting group that is not labile under conditions encountered during polynucleotide synthesis, base deprotection and applications work. The protecting group on the cleavage oxygen is selected so that it may be selectively removed at a desired time under appropriate conditions. The polynucleotide bearing the cleavage oxygen is subjected to these conditions for a time sufficient to cause the protecting group on the cleavage oxygen to be removed, while leaving the protecting groups on the 2'-oxygenated ribonucleotides in the full-length sequence in place. The polynucleotide can then be subjected to basic conditions for a time sufficient to cause cleavage of the phosphodiester bond which links the ribonucleoside the full length sequence, yielding substantially pure, unmodified, full-length chemically synthesized polynucleotides.

In a preferred embodiment, when the polynucleotide is DNA, the preferred protecting group for the cleavage oxygen $\mathbf{R}^1$ or $\mathbf{R}^8$ is t-butyldimethylsilyl. The protecting group t-butylmethylsilyl is stable in the presence of base. t-Butylmethylsilyl may be removed from the cleavage oxygen in the presence of fluoride ion (see (2) in Cleavage Reaction I). Fluoride is not normally present under conditions encountered in DNA synthesis or DNA applications. Other protecting groups may also be suitable for protection of the cleavage oxygen, for example, O-allyl or O-methyl (Greene and Wuts, *Protective Groups in Organic Synthesis*, 2nd Ed., John Wiley and Sons, New York, New York, (1991)) or 2'-O-[1-(2-fluorophenyl)-4-methoxypiperidin-4-yl] (Beijer, et al., *Nucleic Acids Research*, 18 (17): 5143-5151 (1991)) and would be known to one skilled in the art. In another embodiment, when the polynucleotide to which the functional group is reversibly attached is RNA or a DNA/RNA hybrid, the cleavage oxygen is distinguished from all other 2'-oxygen atoms on the ribose ring by the attachment of a protecting group to the cleavage oxygen that is different from the protecting group placed on all other 2'-oxygens, and which requires different conditions for removal. At a desired time, the cleavage oxygen Protecting group is removed, all other 2'-oxygens remain protected, and only the cleavage oxygen is susceptible to chemical cleavage under alkaline conditions. For example, the acid-labile protecting group O-allyl may be used to protect the 2'-oxygens of ribonucleotides of RNA or an RNA/DNA hybrid, and t-butyldimethylsilyl, which may be removed in the presence of fluoride ions, may be used to protect the cleavage oxygen. O-allyl is not removed in the presence of fluoride ion, and may later be removed under acidic conditions.

In the art, RNA phosphoramidites that are commercially available contain t-butyldimethylsilyl protecting groups on the 2'-oxygen of the ribose ring. In order to construct oligomers using the reagent of the present invention and such commercially available RNA phosphoramidite reagents, it would be necessary to place different protecting groups on the 2'-oxygen atoms of the RNA bases, so that only the cleavage oxygen is deprotected prior to exposure to alkaline conditions. Alternatively, an acid-labile protecting group could be placed on the cleavage oxygen, and the RNA bases could be protected with t-butyldimethylsilyl. Selection of appropriate protecting groups for RNA or DNA/RNA hybrid molecules would be known to one skilled in the art.

## Types of Polynucleotides

Although the product of chemical synthesis is single-stranded polynucleotides, base pairing between complementary single-stranded polynucleotide sequences and other single-stranded polynucleotides may occur to form double-stranded polynucleotides. The complementary strand to a chemically synthesized polynucleotide may be another chemically-synthesized polynucleotide, an enzymatically synthesized polynucleotide, or may be a naturally-occurring polynucleotide. A complementary polynucleotide may be composed of sequences that are covalently joined hybrids of chemically synthesized and naturally-occurring polynucleotides. Accordingly, one or more 2'-oxygenated functionalized reagents described herein may be incorporated into polynucleotides in any of the above-mentioned combinations.

## Functional Groups

The present invention contemplates the reversible attachment of substantially any functional group that may be used for detection or for purification of polynucleotides, wherein removal of the functional group by chemical cleavage renders the polynucleotides biologically active by generation of a hydroxyl group at carbon 5 of the 5' terminus.

In a preferred embodiment, the functional group is attached to the reagent molecule before automated synthesis. Alternatively, the functional group may be attached to the polynucleotide sequence after automated synthesis at a site on the sequence derived from the reagent molecule. In the present invention, the preferred functional group is biotin. Biotinylated, chemically-synthesized polynucleotides may be purified from nonbiotinylated failure sequences using avidin affinity chromatography. Additionally, the present invention also contemplates the attachment of other groups useful for purification such as lipoic acid; 2-(4-hydroxyphenylazo)benzoic acid; antisense oligonucleotide sequences, such as oligodeoxyadeonsine, oligodeoxythymidine; or other sequences that are antisense to particular coding or regulatory sequences; antigenic molecules; and the like.

When functional groups that may be used for purification are attached to polynucleotide sequences, affinity media containing a ligand appropriate for attachment of the functional group may be used to purify the functionalized polynucleotides. Examples of affinity ligands include avidin, biotin, antigen-binding portions of antibody molecules, oligonucleotide sequences such as oligodeoxyadenosine, oligodeoxythymidine, antisense sequences to particular coding or regulatory sequences, and the like. These ligands are typically attached to chromatography resins, membranes, or other solid supports. The selection and use of affinity media appropriate for binding of the functional group on the polynucleotide sequence to be purified would be known to one skilled in the art.

The present invention further contemplates the attachment of functional groups that may be used for detection of polynucleotides, such as biotin, 2-(4-hydroxyphenylazo)benzoic acid, trityloxy, methoxytrityloxymethyl, dimethoxytrityloxymethyl, fluorscein, rhodamine, Texas Red, pyrene, digoxygenin, 9-fluorenylmethoxy-carbonyl (Fmoc), isoluminol, a fluorescent lanthanide ion complex, a paramagnetic spin label, an electron-dense label such as gold or ferritin, an enzyme, such as alkaline phosphatase or horseradish peroxidase, or the like.

Using the reagents described herein, more than one functional group may be included in the same reagent molecule. For example, one reagent molecule may include both biotin and dimethoxytrityloxymethyl. The inclusion of more than one functional group in a DNA-functionalizing reagent is known to those skilled in the art.

It is preferred that the functional group be attached directly to the ribonucleoside phosphoramidite reagent, generating the molecule illustrated in (1) of the Cleavage Reaction I above. In an alternative embodiment, a functional group may be added to a chemically synthesized polynucleotide at a position 5' to an $\alpha$-hydroxy phosphodiester bond formed by the incorporation of at least one non-functionalized 2'-oxygenated phosphoramidite reagent. Examples of such non-functionalized 2'-oxygenated phosphoramidites may be selected from the group consisting of reagents derived from ribose. Additionally, nonfunctionalized phosphorylated *cis*- vicinal diols derived from hexose may also be used.

## Synthesis of the Reagent

The phosphoramidite reagent of this invention can be obtained by a series of chemical reactions beginning with a non-protected ribonucleoside, such as uridine. The three hydroxyl moieties of uridine are selectively protected with suitable protecting groups, as follows. Uridine is dissolved in a polar, aprotic solvent, such as pyridine, and treated with monomethoxytrityl chloride, or some other suitable 5'-hydroxyl

tritylating agent and stirred at room temperature for several hours, preferably overnight. The resultant 5'-O-monomethoxytrityl uridine is isolated and then dissolved in a polar, aprotic solvent, such as N,N-dimethylformamide, and treated with imidazole and t-butyldimethylsilyl chloride, or some other suitable 2'- and 3'- hydroxyl silylating reagent according to standard hydroxyl group silylating reaction conditions that would be utilized by one skilled in the art. The reaction mixture is stirred at room temperature for several hours, preferably overnight.

The fully protected ribonucleoside, 2',3'-O-bis(t-butyldimethyl-silyl)-5'-O-monomethoxytrityl uridine, is isolated and further treated to activate the 4-position of the heterocyclic ring for the attachment of a spacer chain. The activation of this position and spacer chain attachment is achieved by adapting a procedure described by Sproat, et al. (*Nucleic Acids Research*, 17(9):3373 (1989)). A suitable spacer chain is one that separates the functional group, from the ribonucleoside portion of the reagent after it is attached to a synthetic oligonucleotide, at a distance sufficient to allow efficient complexation of the functional group when brought into contact with an affinity ligand. An example is the complex biotin forms with avidin or streptavidin.

2',3'-O-Bis(t-butyldimethylsilyl)-5'-O-monomethoxytrityl uridine is treated with triethylamine and N,N-dimethylamino-pyridine, followed by mesitylenesulphonyl chloride in dichloroethane. The intermediate mesitylenesulphonyl derivative is isolated after a reaction time of one hour. The intermediate is dissolved in dichloroethane and treated with triethylamine and 1,8-diazabicyclo- [5.4.0]undec-7-ene followed by 2-nitrophenol. The resultant 2',3'-O-bis(t-butyldimethylsilyl)-5'-O-monomethoxytrityl-4-O-(2-nitrophenyl) uridine is isolated and subsequently treated with triethylamine and 1,6-diaminohexane. The desired 2',3'-O-Bis(t-butyldimethylsilyl)- 5'-O-monomethoxytrityl-4-N-(6-N-aminohexyl)cytidine is isolated from the reaction mixture and this material incorporates the 6-aminohexyl moiety as the spacer chain. The functional group biotin is then attached to the amine terminus of the spacer chain using any standard acid group to amine group coupling chemistry. In this case, the above isolated 2',3'-O-Bis(t-butyldimethylsilyl)-5'-O-monomethoxytrityl-4-N-(6-N-aminohexyl) cyti-dine is dissolved in an appropriate solvent, such as N,N-dimethyl-formamide and treated with 1-hydroxybenzotriazole hydrate, biotin and N-ethyl-N'-3-dimethylaminopropylcarbodiimide hydrochloride, and then held at room temperature for several hours. The desired biotinylated ribose derivative [2',3'-O-bis(t-butyldimethylsilyl)-5'-O-monomethoxytrityl-4-N-(6-N-biotinylaminohexyl)cytidine] is isolated from the reaction mixture.

The silyl protecting groups are then removed from both the 2'- and the 3'-hydroxyl moieties of the ribose ring by standard treatment with tetrabutylammonium fluoride in tetrahydrofuran. The resultant 2',3'-dihydroxy compound is subjected to treatment with imidazole and an appropriate amount of t-butyldimethylsilyl chloride in N,N-dimethylformamide, under standard conditions, in order to prepare the monosilylated derivatives. A regioisomeric mixture of 2'-O-t-butyldimethylsilyl-5'-O-monomethoxytrityl-4-N-(6-N-biotinylaminohexyl)cytidine and 3'-O-t-butyldimethylsilyl-5'-O-monomethoxytrityl-4-N-(6-N-biotinylaminohexyl)-cytidine is isolated and converted directly into the corresponding regioisomeric mixture of 3'- and 2'-O-(2-cyanoethyl N,N-diisopropylphosphoramidites. The phosphoramidites are prepared according to standard procedures known to those skilled in the art. In this case, 2-cyanoethyl N,N-diisopropylchlorophosphoramidite is added to a mixture of the monosilylated ribose derivatives along with diisopropylethylamine and 4-N,N-dimethylaminopyridine in methylene chloride. The resultant regioisomeric mixture of desired phosphoramidites are isolated chromatographically from the crude reaction mixture and are utilized as a mixture to functionalize synthetic oligonucleotides during automated synthesis.

## Chemical Synthesis of Polynucleotides

Automated polynucleotide synthesis is carried out by procedures well-known in the art. The preferred method for polynucleotide synthesis is based upon phosphoramidite chemistry (*Oligonucleotide Synthesis with β−Cyanoethyl Phosphoramidites*, Applied Biosystems User Bulletin, Issue No. 36; July 1, 1986; Andrus A., RNA *Synthesis*, Applied Biosystems User Bulletin, Issue No. 47, April 18, 1988; Narang, SA, Ed., *Synthesis and Applications of RNA and DNA*, Academic Press, San Diego, CA USA (1987)). Although phosphoramidite chemistry is preferred because the efficiency of nucleotide coupling is higher for phosphoramidite chemistry than for phosphodiester or phosphotriester chemistries, the use of phosphodiester or phosphotriester chemistry would be known to one skilled in the art. Phosphoramidite chemistry may be used to synthesize polynucleotides consisting of deoxyribonucleotides (DNA), ribonucleotides (RNA) or hybrid polynucleotides of both DNA and RNA.

In the preferred embodiment of the present invention, a biotinylated 2'-oxygenated phosphoramidite reagent is attached to a polynucleotide sequence by automated synthesis. Additionally, biotin ester may be added in one or more manual steps after automated synthesis to a derivative of a reagent bearing a

reactive group, the reagent having been added to the polynucleotide during automated synthesis. Various strategies for adding biotin to the 5' termini of chemically synthesized polynucleotides in solution would be known to one skilled in the art.

Briefly, chemical synthesis of polynucleotides is carried out as follows: the first nucleoside base of the sequence is attached at the 3' position of a deoxyribose or a ribose ring to an inert support, usually controlled pore glass. The 5' hydroxyl of the deoxyribose or ribose ring is generally protected by a dimethoxytrityl (DMT) group. The first step, detritylation, removes the acid-labile trityl group from the 5' position of the first base, **D**. The second step, coupling, adds the next nucleoside base, **D+1**, to the 5' hydroxyl of nucleoside **D**. The coupling step is normally 98 ±0.5 % efficient, and coupling does not occur at all reactive 5' hydroxyl sites. Therefore, in a third capping step, any unreacted 5' hydroxyl groups are acylated to terminate further addition to those sequences to prevent addition of nucleotides out of proper sequence order. The capped sequences are referred to as failure sequences. In a fourth step, the trivalent phosphite triester bond between the two nucleotides **D** and **D+1** is oxidized, generating pentavalent phosphorous. These four synthesis steps are repeated until the desired sequence is generated.

During solid-phase chemical synthesis, the stepwise yield at each successive nucleotide addition step using phosphoramidite chemistry is optimally 98 ± 0.5%. The sequences to which the incoming nucleotide base is not added are referred to as failure sequences. The 5' termini of failure sequences are chemically modified by a capping reaction using acetic anhydride to prevent further chain elongation of the failures during succeeding addition cycles. Therefore, at the termination of a synthesis program, the polynucleotide composition generated during the solid-phase synthesis is a mixture of full-length sequences and of failure sequences of various lengths.

In order to obtain a purified preparation of the desired full-length sequences, removal of failure sequences is routinely achieved by performing one or more purification steps. Procedures that may be used include reverse phase chromatography, ion exchange chromatography and polyacrylamide gel electrophoresis. Typically, purification methods are more difficult and time-consuming than the actual synthesis (Applied Biosystems, Inc. User Bulletin, Issue No. 13, Revised April 1, 1987), in part because failure sequences do not differ substantially in chemical character from full-length sequences. Thus, the utility of separations based upon chemical character, such as ion exchange or reverse phase chromatography is limited since resolution of full-length sequences from failure sequences decreases as the length of the full-length polynucleotide sequence increases. Additionally, the percentage of failure sequences in the chemically synthesized mixture increases as sequence length increases.

Ion exchange chromatography may be used to separate polynucleotides of different lengths (to about 70-mers) based upon overall sequence charge; however, secondary structure must be minimized by the use of strong denaturants, such as formamide, since phosphate groups that are masked by secondary structure will interfere with the separation of sequences of similar length (Applied Biosystems Inc. User Bulletin Issue, No. 13, Revised April 1, 1987).

Reverse phase chromatography may also be used to purify tritylated or nontritylated oligomers of 50 nucleotides or less. In general, the limitations of both reverse phase chromatography and ion exchange chromatography relate to sequence length and to the degree of secondary structure present in the polynucleotide mixture. Steric problems prevent longer oligonucleotides from entering the pores of the reverse phase matrix, and secondary structure can mask differences in hydrophobicity between desired and unwanted sequences, making separation impossible (Applied Biosystems, Inc. User Bulletin, Issue No. 13, Revised April 1, 1987).

Polyacrylamide gel electrophoresis is a widely used technique that may be used to resolve full-length sequences from failure sequences. Denaturing polyacrylamide gel electrophoresis separates polynucleotides according to sequence length. Electrophoretic resolution of oligomers of more than 70 nucleotides may be achieved; however, the ability to resolve and separate full-length products from longer failure sequences decreases when the synthesis products are long (>70-mers), or when the overall yield of the synthesis is less than optimally efficient. For example, if each cycle of the synthesis of a 50-residue oligonucleotide is 98.5% efficient, full-length products will represent at most 47.6% of the sequences produced by the synthesis [$(.985)^{50}$ = 47.6%]. In order to purify the full-length 50-mer from failure sequences, it is necessary to both resolve the sequence from the longer failure sequences and to recover the sequence by cutting and extracting it from the gel matrix. The process of preparing acrylamide gels and recovering DNA from them is a time-consuming, multi-step procedure that requires the use of highly toxic reagents. In general, gel electrophoresis is an inefficient method for recovering DNA sequences in good yield, especially as sequence length increases.

When synthesis is complete, all sequences, both full-length and failure, are cleaved from the solid support with an ammonia reagent. Protecting groups present on the phosphorous linkage and on the

heterocyclic bases are removed, typically by incubation overnight in an ammonia reagent at temperatures between 55°C and 60°C. One or more purification steps may subsequently be carried out.

Functionalized reagents may be incorporated into chemically synthesized DNA, RNA or DNA/RNA hybrids. Depending upon the chemical structure of the reagent, one or more functional groups may be inserted within the polynucleotide sequence, or alternatively, may be attached at the 5' terminus. Depending upon the chemical structure of the reagent, one or more reagent molecules may be incorporated in sequence, or one or more reagent molecules may be inserted nonsequentially within the polynucleotide sequence.

Another means for polynucleotide purification is chemical modification by addition of a functional group to the polynucleotides to be purified. One or more functional groups may be attached to a chemically synthesized polynucleotide sequence to confer the chemical property of the functional group(s) (J. Goodchild, *Bioconjugate Chemistry* 1(3):165-187 (1990); E.T. Diamandis and T. Christopoulos, *Clin. Chem.* 37(5):625-636 (1991); R.H. Symons, Ed., *Nucleic Acid Probes*, CRC Press, Boca Raton, FL, (1989)). Detection or isolation of functionalized polynucleotides may be carried out based upon the chemical properties of the functional group. For example, the affinity ligand biotin may be covalently attached to chemically synthesized polynucleotides. The biotinylated polynucleotides can be detected using *in vitro* assay methods known in the art, for example, in hybridization assays. The presence of biotin may also be used to advantage to purify polynucleotides of interest, using separation methods based upon the affinity of the ligand biotin for the proteins avidin and streptavidin.

## Purification of Chemically Synthesized Polynucleotides Using Affinity Separation Techniques

When a functional group attached to a chemically synthesized polynucleotide is a ligand with an affinity for a specific binding partner, affinity chromatography may be used to selectively recover functionalized polynucleotides from mixtures of functionalized and nonfunctionalized molecules based upon the specific binding, or affinity, of the ligand and its binding partner. Affinity systems include, but are not limited to, the binding of substrates or binding partners to ligands, antibodies to antigens and sense polynucleotide sequences to their complementary, or antisense polynucleotide sequences, or the like. Examples of suitable supports for affinity separations include, but are not limited to, gels, plastics, glass, magnetized materials, cellulosics, modified sugars, various composites of these and other materials, or the like.

In a preferred embodiment, the ligand biotin may be used for polynucleotide purification. The exceptionally high affinity of biotin for the proteins avidin and streptavidin (Green, N.M., *Advances in Protein Chemistry*, Vol 9, pp 85-133 (1975)), Wilchek M. and Bayer E.A, *Analytical Biochem*, 171:1-32 (1988); Bayer E.A. and Wilchek M., *Methods in Biochem Anal*, 26:1-45 (1980)), has resulted in the development of numerous applications based upon this highly specific interaction, (*Nucleic Acid Probes*, R.H. Symons, Ed., CRC Press, Inc., Boca Raton, FL (1989)).

Purification of biotinylated full-length polynucleotides by avidin attached to solid supports is independent of full-length sequence concentration, length and secondary structure. It is known that one or more of these constraints may limit the usefulness of existing methods, for examples, reverse phase chromatography, ion exchange chromatography or denaturing polyacrylamide electrophoresis.

Avidin monomer (EP 423938, Affinity Chromatography Media- Comprising Avidin Monomer Covalently Linked to Insoluble Support, filed) is the preferred affinity medium, as biotin binding to avidin monomer is readily reversible under physiological conditions and permits recovery of highly pure biotinylated polynucleotides in high yield. EP 423938 is incorporated herein by reference. Additionally, avidin tetramer, streptavidin tetramer or streptavidin monomer may also be used. Binding of biotin to tetrameric avidin and to tetrameric streptavidin is generally observed to be irreversible. Therefore an advantage of using avidin monomer is that quantitative recovery of the desired biotinylated molecules may be obtained by elution of those molecules away from the avidin monomer using biotin.

To purify full-length biotinylated polynucleotides, the crude synthesis mixture of biotinylated full-length sequences and nonbiotinylated failure sequences is reconstituted in water or in an appropriate buffer. Examples of buffers include sodium or potassium phosphate buffer, tris[hydroxymethyl]aminomethane or ammonium acetate buffer, or the like, prepared at concentrations 10 to 100 millimolar of between pH 6 and pH 8. Other conditions favorable for reconstitution of polynucleotide preparations may also be used, and these conditions would be known to one skilled in the art.

Purification of biotinylated sequences away from nonbiotinylated failure sequences may be accomplished by passing the reconstituted synthesis mixture into contact with avidin fixed to a solid support. Then, the support is washed thoroughly with water or an appropriate buffer to remove all nonbound failure sequences. The affinity of biotin for avidin results in selective retention of the biotinylated sequences in

contact with the avidin, while nonbiotinylated failure sequences are not retained in contact with the avidin. Biotinylated polynucleotides in high yield and high purity may be eluted from the avidin monomer medium using a solution of free biotin. Biotin of 2 to 20 millimolar dissolved in an appropriate buffer, for example, potassium or sodium phosphate buffer, or ammonium acetate buffer, at a concentration of 10 millimolar to 500 millimolar, and at a pH of 5 to 8 may be used.

## Recovery of Biologically Active Polynucleotides by α- or β-Hydroxy Phosphodiester Bond Hydrolysis

After biotinylated full-length polynucleotides are eluted from the avidin affinity support, biologically active polynucleotides may be generated by chemical cleavage of the functional group from the 5' terminus of the polynucleotide sequence.

Further purification of the cleavage reaction products may be performed to remove reaction by-products by subjecting the cleavage reaction mixture to avidin affinity chromatography, ion exchange or reverse phase chromatography, or denaturing polyacrylamide gel electrophoresis.

Optionally, chemical cleavage may be performed while the biotinylated polynucleotides are still attached to the avidin affinity support, after nonbiotinylated failure sequences have been removed, but before the biotinylated polynucleotides are eluted away from the avidin support using biotin.

## Uses

Functionalized chemically synthesized DNA and RNA may be used as probes for a variety of applications including detection of polynucleotides of interest and purification of polynucleotides from complex mixtures *in vivo* or *in situ* including Southern analysis, Northern analysis, polymerase chain reaction, polynucleotide sequencing, histochemical detection of gene expression products, recovery of desired gene fragments for cloning, and other procedures well known in the art (Symons, R.H., *Nucleic Acid Probes*, CRC Press, Inc., Boca Raton, FL; Conn, P.M. Ed., *Gene Probes, Methods in Neurosciences*, Vol. 1 Academic Press, San Diego, CA (1989)).

Biotinylated polynucleotides may be used for isolation of complementary polynucleotides from complex mixtures, for example, by hybridization of the desired sequence with the complementary chemically synthesized biotinylated sequence, followed by avidin affinity chromatography to separate the desired sequence from unwanted components in the mixture.

Additionally, a biotinylated terminus may serve as a binding site for attachment of single-stranded polynucleotides to an avidin-bearing support, such as a magnetized resin or a membrane, and enzymatic or chemical modification of the immobilized polynucleotide may be carried out. For example, cloning reactions, including complementary polynucleotide strand synthesis and solid-phase sequencing reactions may be carried out while the polynucleotide sequence is immobilized on avidin. An advantage of immobilization is that between modification steps, excess reagents and buffers may be washed away from the immobilized polynucleotides without precipitation or addition of organic solvents before proceeding to the next reaction step. A further advantage of attaching biotin to a chemically synthesized polynucleotide through a hydrolyzable bond is that once all desired modifications have been made to an immobilized polynucleotide, the biotin may be chemically cleaved, to generate a biologically active polynucleotide.

In order to more fully illustrate the nature of this invention and the manner of practicing the same, the following examples are presented.

## General Procedures

The following examples were carried out using one or more of the general procedures set forth below.

### Procedure 1. Automated Synthesis of Polynucleotides

Chemical synthesis of polynucleotides was performed on an Applied Biosystems Model 394 DNA/RNA Synthesizer. RNA dimethoxytrityl (DMT)-cyanoethyl phosphoramidites were purchased from Peninsula Labs (Belmont, CA, USA). Biotin dX was purchased from Midland Chemical Company (Midland, TX). Low-water acetonitrile was purchased from J.T. Baker (Phillipsburg, NJ), and ammonium hydroxide was purchased from Pfaltz and Bauer (Waterbury, CT). DMT-cyanoethyl DNA phosphoramidites and all other synthesis reagents were purchased from Applied Biosystems (Foster City, CA), prepared and used according to procedures recommended by the manufacturer. Software programs used were either DMT-Off, Auto [end RNA] or DMT-On, manual chemical synthesis as described in the Applied Biosystems Model 394 DNA/RNA

synthesizer User Manual. Additional information for RNA synthesis cycles were obtained from Applied Biosystems User Bulletin, Issue No. 47 (A. Andrus, RNA Synthesis, April 18, 1988) and Applied Biosystems User Bulletin, Issue No. 53, (*RNA Synthesis With DMT−Cyanoethyl RNA Phosphoramidites*, December 1, 1989). The lengthened coupling cycle times described in User Bulletin, Issue No. 53 to optimize coupling of ribonucleoside phosphoramidites were routinely used for all synthesis cycles, including DNA synthesis. Nonbiotinylated polynucleotides were prepared by a single DMT-Off, Auto [end RNA] synthesis program.

To prepare biotinylated polynucleotides, two separate synthesis programs were used. In the first synthesis program, the full-length polynucleotide sequence minus the biotinylating reagent was synthesized, as a Trityl-On Manual synthesis, to leave the trityl protecting group attached to the 5' termini of full-length sequences. The sequences were not cleaved from the synthesis support, and the columns remained in place on the DNA synthesizer for the second synthesis program. In the second synthesis program, the appropriate biotinylating reagent was added to the 5' terminus of full-length sequences. To do this, the DNA synthesizer was programmed to perform a DMT-Off, Auto [end RNA] synthesis program consisting of a single cycle. The tritylated polynucleotides from the first synthesis served as the starting material for the single addition cycle of program 2, in which one molecule of reagent bearing biotin was added to the 5' termini of full-length sequences.

Alter all synthesis steps were completed, polynucleotides were manually cleaved from controlled pore glass supports using the double-syringe method described in Applied Biosystems User Bulletin 53 (pp. 4-5), using a fresh solution of 3:1 (v:v) ammonium hydroxide:ethanol. Heterocyclic base protecting groups and phosphate protecting groups were removed by further incubation for 12 to 18 hours in the same ammonium hydroxide:ethanol solution in a 55°C water bath. Samples were then freeze-dried in a Speed-Vac Vacuum Concentrator (Savant Instrument Company, Farmingdale, NY). The dried samples were reconstituted in 1 milliliter of water and freeze-dried again. Reconstitution with 1 milliliter of water and freeze-drying were repeated.

Stepwise and overall chemical synthesis yields were determined by spectrophotometric determination of the amount of monomethoxy- or dimethoxytrityl ions released at each synthesis step. The trityl assay procedures used are described as Procedure 1 in Applied Biosystems User Bulletin No. 13-Revised (*The Evaluation and Purification of Synthetic Oligonucleotides*, April 1, 1987). The trityl assay measures the number of micromoles of trityl ion collected after each addition cycle, which is equivalent to the number of micromoles of nucleotide coupled during that same cycle. For a one micromole scale synthesis, each trityl collected was diluted to 8 milliliters with 0.1 molar toluene sulfonic acid (monohydrate) in acetonitrile. After thorough mixing with a vortex mixer, 150 microliters of this solution was diluted into 2.75 milliliters of 0.1 molar toluene sulfonic acid in acetonitrile in a 1-centimeter path length cuvette. This series provides a 19.33-fold dilution of the original trityl ion collected. Micromoles of dimethoxytrityl ion were determined by reading the absorbance of each sample at 498 nanometers (the extinction coefficient e = 70 micromoles$^{-1}$centimeter$^{-1}$milliliter), and the release of the monomethoxytrityl cation was monitored at 478 nanometers (for monomethoxytrityl, the extinction coefficient is 56 micromoles$^{1}$-centimeter$^{-1}$milliliter. Calculations of trityl ion concentrations were determined by the equation millimoles of trityl ion = (absorbance) (dilution factor) (volume)/extinction coefficient. Stepwise yields were calculated by dividing the micromoles of trityl ion released for step **d** by the micromoles of trityl ion released for step **d-1**, where **d** is a nucleoside addition step. The overall synthesis yield is calculated as the cumulative sum of the stepwise yields X 100.

Procedure 2. Chemical Cleavage of α-Hydroxyphosphodiester Bonds

The polynucleotide sample was suspended in a microcentrifuge tube to a volume of 125 microliters of avidin monomer affinity chromatography elution buffer or water. To remove the silyl protecting group, 100 to 300 microliters of 1 molar tetrabutyl- ammonium fluoride in tetrahydrofuran (TBAF/THF) (Aldrich Chemical Company, Milwaukee, WI) was added to the polynucleotide sample (Scaringe, et al., *Nucleic Acids Research*, 18(18):5433-5441 (1990)). The sample was mixed thoroughly and incubated at room temperature (18°-25°C) for 12 to 24 hours. Any precipitate formed during the incubation in TBAF/THF was removed by centrifuging the sample in a Jouan MR-14.11 microcentrifuge, and the supernatant was transferred to a clean microcentrifuge tube. To initiate the cleavage procedure, an equal volume of 1 normal sodium hydroxide was added to the mixture, the sample was mixed, then was incubated at room temperature (18 to 25 degrees centigrade) for 4 to 24 hours. The volume of the sample was measured, and a volume of 0.05 molar ammonium acetate equal to 1.5 times the volume of the cleavage reaction mixture was added to the mixture. Any precipitate that formed was removed by microcentrifugation.

Procedure 3. Avidin Monomer Affinity Chromatography

Dried, crude mixtures of chemically synthesized polynucleotides were resuspended in water at a concentration of 20 to 30 micrograms/ microliter. 250 microliters of ImmunoPure® Monomeric Avidin (Pierce, Rockford, IL) resin slurry was packed into Pierce Polystyrene Columns (Pierce, Rockford, IL) using methods recommended by the manufacturer, to yield a resin bed 8 millimeters high X 6 millimeters wide. Procedures used to load, wash and elute the polynucleotide sample were the same as those recommended by the manufacturer for protein purification, and were supplied with the ImmunoPure® avidin resin. Prior to sample loading, the resin was equilibrated in phosphate-buffered saline using gravity flow. After sample loading, the column was washed with at least ten column volumes of the same buffer so that all nonbound substances were washed from the resin. To elute biotinylated polynucleotides, at least ten column volumes of the 2 millimolar Biotin Blocking and Elution Buffer supplied with the resin was passed through the column to elute biotinylated polynucleotides from the column. 250 microliter to 1 milliliter fractions were collected. The recoveries of nonbiotinylated and biotinylated polynucleotides from the column chromatography were determined by measuring absorbance of flowthrough and eluted fractions at 260 nanometers on a Gilford Response™ spectrophotometer using the method described by Ausubel, et al. (*Current Protocols in Molecular Biology.*, Vol. 1, Appendix 3, pp 9-10, John Wiley & Sons, New York, NY (1989)).

Procedure 4. Desalting Using a Hand-Held C18-Reverse-Phase Silica Gel Column

Oligonucleotides were desalted using the procedure described by Sambrook, et al. (*Molecular Cloning: A Laboratory Manual*, 2nd Edition, Vol. 2, Chapter 11, p. 11.29 to 11.30, Cold Spring Harbor Press, Cold Spring Harbor, New York, NY (1989)).

Procedure 5. Polyacrylamide Gel Electrophoresis of Polynucleotides

The polynucleotide products of chemical synthesis, avidin monomer affinity chromatography purification, and chemical cleavage was examined by separation on 20% acrylamide gels containing 7 molar urea (Ausubel, et al., (*Current Protocols in Molecular Biology*, Vol 1, Unit 2.12.1 - 2.12.5, John Wiley & Sons, NY (1989)). Acrylamide (40% solution of 29:1 acrylamide:bisacrylamide) was purchased from Fisher Scientific (Pittsburgh PA). Urea, N,N,N',N'-tetramethylethylenediamine (TEMED), and ammonium persulfate were purchased from Sigma Chemical Company (St. Louis, MO) and 10X tris-borate-EDTA buffer (Marathon Gel-Mix Running Mate 10X TBE Buffer Concentrate) were purchased from Bethesda Research Laboratories (Bethesda, MD). Gels were cast in 7.5 centimeter X 7.5 centimeter by 0.5 millimeter cassettes purchased from Novex (San Diego, CA). Single-stranded DNA was examined by staining with ethidium bromide (Sigma Chemical Company St. Louis, MO) and illuminated using shortwave ultraviolet light (Sambrook. et al., *Molecular Cloning, a Laboratory Manual*, Second Edition, Volume 1, page 6.44 (1989)).

Procedure 6. Polyacrylamide Gel Retardation Assay for Biotinylated Polynucleotides

Cleaved and noncleaved polynucleotides were examined for the presence of biotin by mixing the samples with streptavidin prior to running on polyacrylamide gels according to Procedure 5. The migration of biotinylated polynucleotides through acrylamide gels is substantially retarded when the polynucleotides are bound to streptavidin. Streptavidin was suspended in water at a concentration of one microgram per milliliter. A mixture of polynucleotide in water and streptavidin in water was mixed in the ratio of one microgram polynucleotide to one microgram streptavidin and incubated at room temperature for approximately one minute. Samples were then mixed with an equal volume of formamide loading buffer (Ausubel. et al., *Current Protocols in Molecular Biology*, Vol 1, Unit 2.12.1 - 2.12.5, John Wiley & Sons, New York, NY (1989)) and loaded immediately onto polyacrylamide gels and run according to Procedure 5.

Example 1. Synthesis of 2'-O-t-butyldimethylsilyl-5'-O-mono- methoxytrityl-4-N-(6-N-biotinylaminohexyl)-cytidine-3'-O-(2-cyano-ethyl N,N-diisopropylphosphoramidite) and 3'-O-t-butyldimethylsilyl-5'-O-monomethoxytrityl-4-N-(6-N-biotinylaminohexyl)cytidine-2'-O-(2-cyanoethyl N,N-diisopropyl-phosphoramidite).

All anhydrous solvents were purchased from Aldrich Chemical Company packaged under nitrogen in Sure/seal™ bottles. All other solvents were reagent grade or better. Triethylamine and diisopropylethylamine were dried over KOH. All reactions were carried out in septum-fitted and argon-purged round bottomed flasks and run under an argon atmosphere. All liquid reagents were added via syringe. Thin layer chromatographic (TLC) data ($R_f$ values) were obtained on Merck® Kieselgel 60 $F_{254}$ analytical glass backed sheets. TLC visualization techniques included ultraviolet light (254 nanometers), iodine vapors, concentrated HCl vapors, 0.2 percent dimethylaminocinnamaldehyde in ethanol containing 2 percent $H_2SO_4$. $^1H$ and $^{31}P$ nuclear magnetic resonance (NMR) spectra were obtained on Varian XL-200 and Geol FX-90 spectrometers, respectively. The $^1H$-NMR spectra were referenced to tetramethylsilane (internal). $^{31}P$-NMR chemical shifts quoted are downfield from 85 percent $H_3PO_4$ (external). Flash column chromatography was performed with Merck silica gel, grade 60, 230-400 mesh.

All of the compounds prepared were examined for purity by normal phase silica gel thin layer chromatography (TLC) in a variety of solvent systems, using ultraviolet light for visualization and different reagents (iodine vapors, concentrated HCl vapors, 0.2 percent dimethylaminocinnamaldehyde in ethanol containing 2 percent $H_2SO_4$). The structures were verified by $^1H$-NMR and $^{31}P$-NMR where applicable. In some cases elemental analyses were obtained.

Uridine (5.00 grams, 20.49 millimoles) was dissolved in anhydrous pyridine (75 milliliters) and treated with monomethoxy-trityl chloride (7.59 grams, 24.59 millimoles) and stirred at room temperature for 24 hours. The reaction was quenched with methanol (25 milliliters) and stirred for an additional 30 min. It was then diluted with methylene chloride (250 milliliters) and washed with 200 milliliters of half saturated aqueous sodium bicarbonate solution three times, 200 milliliters of water three times, followed by 200 milliliters of saturated aqueous sodium chloride solution, dried over $Na_2SO_4$ and concentrated. The residue was coevaporated with toluene to remove excess pyridine.

5'-O-Monomethoxytrityluridine (13.89 grams) was obtained as a dry, pale yellow foam product and was used without further purification in the next reaction.

5'-O-Monomethoxytrityluridine (10.59 grams, 20.50 millimoles) was dissolved in anhydrous N,N-dimethylformamide (DMF, 100 milliliters)), treated with imidazole (8.37 grams, 122.94 millimoles) and t-butyldimethylsilyl chloride (9.27 grams, 61.50 millimoles) and stirred at room temperature for 24 hours. The reaction was diluted with diethyl ether (200 milliliters) and washed twice with 200 milliliters half saturated sodium bicarbonate solution, twice with 200 milliliters water, once with 200 milliliters saturated aqueous sodium chloride solution, and dried over $Na_2SO_4$. After concentrating, the residue was dried by co-evaporating with toluene to afford 2',3'-O-bis(t-butyldimethylsilyl)-5'-O-monomethoxytrityl uridine as a pale yellow milky residue (19.41 grams). This material was used in the next reaction without further purification. Dry triethylamine (18.20 milliliters, 130.58 millimoles), N,N-dimethylaminopyridine (0.80 gram, 6.55 millimoles) and mesitylenesulphonyl chloride (7.97 grams, 36.44 millimoles) were added to a stirred solution of 2',3'-O-Bis(t-butyldimethylsilyl)-5'-O-monomethoxytrityl-4-O-(2-nitrophenyl) uridine (19.40 grams, 26.04 millimoles) in anhydrous dichloroethane (150 milliliters). The reaction was stirred at room temperature for 1 hour. The amber colored solution was diluted with methylene chloride (200 milliliters) and then poured, with stirring, into a beaker containing cold saturated aqueous sodium bicarbonate solution (300 milliliters). The mixture was transferred to a separatory funnel, the phases separated, and the aqueous layer was discarded. The organic layer was washed twice with 200 milliliters of half saturated sodium bicarbonate solution, once with 200 milliliters of saturated aqueous sodium chloride solution, dried over $Na_2SO_4$ and concentrated under reduced pressure to afford the intermediate mesitylenesulphonyl derivative as an orange foam (25.29 grams). This material was dissolved in anhydrous 1,2-dichloroethane (100 milliliters) and treated with dry triethylamine (5.90 milliliters, 42.33 millimoles) and 1,8-diazabicyclo[5.4.0]undec-7-ene (0.78 milliliter, 5.22 millimoles), followed by 2-nitrophenol (5.56 grams, 39.97 millimoles) under argon. The mixture was stirred at room temperature for 2 hours, then stored in the refrigerator for several hours. The reaction was diluted with methylene chloride (300 milliliters) and washed three times with 200 milliliters of saturated aqueous sodium bicarbonate solution, once with 200 milliliters of water, twice with 200 milliliters of half saturated sodium bicarbonate solution, once with 200 milliliters saturated sodium chloride solution, and dried over $MgSO_4$. Evaporation and chromatographic purification (using silica gel, and methylene chloride as eluant) provided 2',3'-O-Bis(t-butyldimethylsilyl)-5'O-monomethoxytrityl-4-O-(2-nitrophenyl) uridine (11.19 grams) as the desired product in 63% overall yield beginning from uridine.

2',3'-O-Bis(t-butyldimethylsilyl)-5'-O-monomethoxytrityl-4-O-(2-nitrophenyl) uridine (9.40 grams, 10.85 millimoles) was dissolved in anhydrous acetonitrile (85 milliliters) and dry triethylamine (1.51 milliliters, 10.83 millimoles), followed by 1,6-hexanediamine (12.81 grams, 110.23 millimoles) were added. The solution was stirred overnight under dry argon. The deep yellow mixture was diluted with methylene chloride (250 milliliters) and filtered to remove the precipitated 2-nitrophenol. The filtrate was washed with 200 milliliters of saturated aqueous sodium bicarbonate solution, three times with 200 milliliters of half saturated aqueous sodium bicarbonate solution, three times with 200 milliliters of water, once with 200 milliliters of saturated sodium chloride solution, dried over $MgSO_4$ and concentrated to afford a bright

yellow solid. This material was dissolved in methylene chloride and washed three times with 200 milliliters saturated aqueous sodium chloride solution, dried over $Na_2SO_4$ and $MgSO_4$, and concentrated to afford 2',3'-O-Bis(t-butyldi-methylsilyl)-5'-O-monomethoxytrityl-4-N-(6-N-aminohexyl)cytidine as a bright yellow foam (8.16 grams). This material was sufficiently pure to be used in the next reaction. To a solution of 2',3'-O-Bis(t-butyldi-methylsilyl)-5'-O-monomethoxytrityl-4-N-(6-N-aminohexyl) cytidine (4.96 grams, 5.88 millimoles) in anhydrous N,N-dimethylformamide (35 milliliters), 1-hydroxybenzotriazole hydrate (0.8791 grams, 6.51 millimoles), biotin (1.4542 grams, 5.95 millimoles) and N-ethyl-N'-3-dimethylaminopropylcarbodiimide hydrochloride (1.8349 grams, 9.57 millimoles) were added. The reaction was kept at room temperature, under argon, for 2 hours with stirring. The reaction was diluted with methylene chloride (300 milliliters) and carefully washed three times with 200 milliliters each of 5% aqueous sodium bicarbonate, water, and saturated aqueous sodium chloride, dried over $Na_2SO_4$, filtered, and concentrated under reduced pressure. The resulting clear yellow oil was poured as a thin ribbon into a beaker containing 200 milliliters of rapidly stirring water. The aqueous mixture was twice extracted with diethyl ether. The combined diethyl ether layers were washed once with 200 milliliters water, twice with 100 milliliters of 5% aqueous sodium bicarbonate, once with 100 milliliters of water, once with saturated aqueous sodium chloride, dried over $Na_2SO_4$, filtered and concentrated under reduced pressure. 2',3'-O-Bis(t-butyldimethylsilyl)-5'-O-monomethoxytrityl-4-N-(6-N-biotinylaminohexyl)cytidine was obtained as a pale yellow foam (5.59 grams, 88.9% yield).

2',3'-O-Bis(t-butyldimethylsilyl)-5'-O-monomethoxytrityl-4-N-(6-N-biotinylamin ohexyl)cytidine (5.52 grams, 5.16 millimoles) was dissolved in anhydrous tetrahydrofuran (20 milliliters), treated with 1 molar tetrabutylammonium fluoride in tetrahydrofuran (12.2 milliliters, 12.2 millimoles) and stirred under argon at room temperature. After 90 minutes, the reaction mixture was quenched by the addition of methanol (10 milliliters) and stirred for an additional 10 minutes. The mixture was concentrated to an orange-yellow syrup, redissolved in (3:1:1, v/v/v) pyridine/methanol/water (30 milliliters) and treated with the pyridinium form of Dowex® 50X2-200 ion exchange resin (20 grams). The mixture was stirred for 30 minutes at room temperature and then filtered to remove the resin. The resin was rinsed with the same solvent mixture. The combined rinses and filtrate were concentrated under reduced pressure. The resultant residue was coevaporated three times with absolute ethanol, redissolved in methylene chloride and concentrated under reduced pressure.

5'-O-Monomethoxytrityl-4-N-(6-N-biotinylaminohexyl)cytidine was obtained as a dry yellow foam (3.53 grams, 81.3% yield). 5'-O-Monomethoxytrityl-4-N-(6-N-biotinylaminohexyl)cytidine (1.98 grams, 2.354 millimoles) was dissolved in anhydrous N,N-dimethylforma-mide (10 milliliters), treated with imidazole (0.48836 gram, 7.10 millimoles) and t-butyldimethylsilyl chloride (0.5356 gram, 3.55 millimoles) and stirred at room temperature for 24 hours. The reaction was diluted with methylene chloride and washed with 5% aqueous sodium bicarbonate solution, saturated aqueous sodium chloride solution, dried over $Na_2SO_4$, filtered and concentrated under reduced pressure. A mixture of 2'-O-t-Butyldimethylsilyl-5'-O-mono-methoxytrityl-4-N-(6-N-biotinylaminohexyl)cytid ine and 3'-O-t-Butyldimethylsilyl-5'-O-monomethoxytrityl-4-N-(6-N-biotinylamino-hexyl)cytidine was obtained as a clear yellow syrup (2.45 grams). This material was used in the next reaction without further purification. A mixture of 2'-O-t-Butyldimethylsilyl-5'-O-monomethoxytrityl-4-N-(6-N-biotinylaminohexyl)cytidine and 3'-O-t-Butyldimethylsilyl-5'-O-monomethoxytrityl-4-N-(6-N-biotinylaminohexyl )cytidine (1.82 grams, 1.90 millimoles) was dissolved in anhydrous methylene chloride, then treated with diisopropylethylamine (1.40 milliliters, 8.04 millimoles) and 4-N,N-dimethylaminopyridine (50.2 milligrams, 0.411 millimole). 2-Cyanoethyl N,N-diisopropylchlorophosphoramidite (0.56 milliliter, 2.51 millimoles) was added to this mixture via syringe over 8 minutes. When the addition was complete, the reaction mixture was stirred at room temperature for 24 hours. An additional 45 microliters (0.202 millimole) of 2-cyanoethyl N,N-diisopropylchlorophosphoramidite was added and stirring at room temperature was continued for an additional 19 hours. The reaction mixture was diluted with methylene chloride and washed with 5 percent aqueous sodium bicarbonate, then saturated aqueous sodium chloride, dried over $Na_2SO_4$, filtered and then concentrated under reduced pressure as a dry yellow foam (2.00 grams. crude yield). This material was chromatographed on Silica Gel (50 grams) and eluted with methylene chloride/triethylamine (90/10). The fractions containing desired product, of similar purity, were pooled. A total of 883 milligrams of 2'-O-t-Butyldimethylsilyl-5'O-monomethoxytrityl-4-N-(6-N-biotinylaminohexyl)cytidine-3'-O-(2-cyanoethyl N,N-diisopropylphosphoramidite) and 3'-O-t-Butyldimethylsilyl-5'-O-monomethoxytrityl-4-N-(6-N-biotinylaminohexyl)cytidine-2'-O-(2-cyanoethyl N,N-diisopropyl- phosphoramidite) was obtained (40% yield).

Example 2. Chemical Synthesis of a Biotinylated 25-Base Deoxyribonucleotide Sequence and Chemical Cleavage of Biotin from the 5' Terminus of the Full-Length Deoxyribonucleotide Sequence

A 25 base sequence of the Lambda primer DNA sequence (λ-primer), nucleotides 7131 to 7155 of the lambda genome sequence, (Sanger. et al., *J. Mol. Biol*, 162:729 (1982)) was synthesized in the first synthesis program of Procedure 1. In a second synthesis program as described in Procedure 1, a single biotin moiety derived from a biotinylating reagent composed of a mixture of 2'-O-t-butyldimethyl-silyl-5'-O-monomethoxytrityl-4-N-(6-N-biotinylaminohexyl)cytidine-3'-O-(2-cyanoethyl N,N-diisopropylphosphoramidite) or 3'-O-t-butyldimethylsilyl-5'-O-monomethoxytrityl-4-N-(6-N-biotinylamino-hexyl)cytidine-2'-O-(2-cyanoethyl N,N-diisopropylphosphoramidite) molecule was added to the 5' terminus of the 25-base lambda primer sequence to yield:

NGGTCAACAT GCCTGTGCTT GAGTAG (SEQ ID NO:1)

Stepwise and overall synthesis yields were determined by trityl ion analysis as described in Procedure 1. Heterocyclic base deprotection was carried out as described in Procedure 1. The freeze-dried crude synthesis mixture was reconstituted in water, quantitated by measuring the absorbance of the sample at 260 nanometers as described in Procedure 1.

The silyl protecting group on the cleavage oxygen derived from the 2'-O-t-butyldimethylsilyl-5'-O-monomethoxytrityl-4-N-(6-N-biotinylaminohexyl)cytidi ne-3'-O-(2-cyanoethyl N,N-diisopropylphosphoramidite) or 3'-O-t-butyldimethylsilyl-5'-O-monomethoxytrityl-4-N-(6-N-biotinylaminohexyl )cytidine-2'-O-(2-cyanoethyl N,N-diisopropyl-phosphoramidite) reagent molecule was removed according to Procedure 2 by incubation in 300 microliters of 1 molar tetrabutyl-ammonium fluoride in tetrahydrofuran for 24 hours. Cleavage was accomplished by the addition of 1 normal sodium hydroxide to the reaction mixture followed by incubation for 20 hours at 25 degrees centigrade. After the cleavage reaction was complete, the sample was desalted using Procedure 4 and examined by the streptavidin gel retardation and polyacrylamide gel electrophoresis assays using Procedures 5 and 6. Relative migration of DNA in the gel were expressed as $R_f$. $R_f$ refers to the relative position of the DNA band in the gel, with $R_f$ of 0 being no migration, and $R_f$ of 1.0 being migration to the bottom edge of the gel. Larger molecules have lower relative $R_f$ values. Nonbiotinylated DNA of 25 bases in length migrated to an $R_f$ value of 0.95 in the gel matrix. A DNA band having an $R_f$ value of 0.37 appeared in uncleaved SEQ ID NO:1 samples that were mixed with streptavidin prior to electrophoresis. The band with $R_f$ 0.37 was absent in SEQ ID NO:1 samples that were subjected to a cleavage reaction prior to mixing with streptavidin.

Example 3. Chemical Synthesis of a Biotinylated 25-Base Deoxyribonucleotide Sequence Having One or Three Ribonucleotide Bases Within the Sequence and a Biotin Moiety Derived from a Noncleavable Reagent at the 5' Terminus, and Chemical Cleavage of Biotin From the 5' Terminus of the Full-Length Deoxyribonucleotide Sequence

Uridine and a commercial, noncleavable biotinylating reagent were attached to the 5' terminus of the lambda primer DNA sequence to create the sequences:

NUUUGGTCAA CATGCCTGTG CTTGAGTAG (SEQ ID NO:2), and

NUGGTCAACA TGCCTGTGCT TGAGTAG (SEQ ID NO:3)

wherein N is biotin dX (N-biotinyl-aminopropyl 2-cyanoethyl N,N-diisopropylphosphoramidite), a commercial biotinylating reagent purchased from Midland Chemical Company (Midland, Texas), and U is the ribonucleoside uridine. Synthesis program 1 of Procedure 1 was used to synthesize a 26 nucleotide sequence of SEQ ID NO:2 and synthesis program 1 was used to synthesize the first 28 nucleotides of SEQ ID NO:3. Synthesis program 2 of Procedure 1 was then used to add one molecule of biotin reagent to the 5' termini of each of SEQ ID NO:2 and SEQ ID NO:3. The freeze-dried crude polynucleotide mixtures were prepared for avidin monomer affinity chromatography as described in Procedure 3 by reconstitution in water

to a concentration of 1 microgram per milliliter. After passing the crude mixture into contact with the avidin resin, the avidin monomer resin was thoroughly washed to remove nonbiotinylated failure sequences, and highly pure, full-length biotinylated polynucleotides of SEQ ID NO:2 and SEQ ID NO:3 were eluted in 250 microliter fractions from the resin using the 2 millimolar Biotin Blocking and Elution buffer, according to Procedure 3. Highly pure full-length polynucleotides were recovered by avidin monomer affinity chromatography using Procedure 3. Biotin was removed from the 5' terminus by $\alpha$-hydroxy phosphodiester bond cleavage using Procedure 2. An equal volume of tetrabutylammonium fluoride in 1 molar tetrahydrofuran (TBAF/THF) was added to each sample to be cleaved. The samples were incubated for 24 hours at 25 degrees centigrade. A volume of 1 normal sodium hydroxide equal to the volume of the elution buffer:TBAF/THF mixture was then added, and the samples were incubated for an additional 18 hours at 25 degrees centigrade. The samples were diluted to 6 milliliters with water and desalted using Procedure 5. Cleavage was observed by PAGE analysis using Procedure 5. For both SEQ ID NO:2 and SEQ ID NO:3, the appearance of major bands at 25 bases and disappearance of larger DNA bands representing biotinylated DNA confirmed that chemical cleavage of biotin from 5' termini was successful, and that the expected 25-base lambda primer was generated.

Samples of SEQ ID NO:2 were additionally analyzed by the gel retardation assay of Procedure 6. Full-length biotinylated SEQ ID NO:2 DNA was observed to migrate to $R_f$ 0.95 in the absence of streptavidin, and to $R_f$ 0.37 in the presence of streptavidin. After chemical cleavage, the biotinylated DNA migrating to $R_f$ 0.37 was reduced to barely detectable levels, and a major band at $R_f$ 0.95, representing cleaved 25-base lambda primer DNA was seen both in the presence and absence of streptavidin, indicating successful cleavage of biotin from the 5' terminus of SEQ ID NO:2.

## SEQUENCE LISTINGS

SEQ ID NO:1

SEQUENCE TYPE: NUCLEIC ACID
MOLECULE TYPE: DNA (GENOMIC)
ORIGINAL SOURCE: BACTERIOPHAGE LAMBDA
SEQUENCE LENGTH: 25 NUCLEOTIDES
TOPOLOGY: LINEAR
STRAND TYPE: SINGLE
DIRECTION: 5' TO 3'
N: a commercial biotinylating reagent as hereinbefore defined
SEQUENCE: N GGTCAACATG CCTGTGCTTG AGTAG

SEQ ID NO:2

SEQUENCE TYPE: NUCLEIC ACID
MOLECULE TYPE: DNA (GENOMIC)
ORIGINAL SOURCE: BACTERIOPHAGE LAMBDA
SEQUENCE LENGTH: 28 NUCLEOTIDES
TOPOLOGY: LINEAR
STRAND TYPE: SINGLE
DIRECTION: 5' TO 3'
N: a commercial biotinylating reagent as hereinbefore defined
SEQUENCE: N UUUGGTCAAC ATGCCTGTGC TTGAGTAG

SEQ ID NO:3

SEQUENCE TYPE: NUCLEIC ACID
MOLECULE TYPE: DNA (GENOMIC)
ORIGINAL SOURCE: BACTERIOPHAGE LAMBDA
SEQUENCE LENGTH: 26 NUCLEOTIDES
TOPOLOGY: LINEAR
STRAND TYPE: SINGLE
DIRECTION: 5' TO 3'
N: a commercial biotinylating reagent as hereinbefore defined
SEQUENCE: N UGGTCAACAT GCCTGTGCTT GAGTAG

## Claims

1. A process comprising a step (a) comprising attaching a functionalized ribonucleoside at any position along a desired full-length chemically synthesized polynucleotide, such as a polydeoxyribonucleotide,

wherein the ribonucleoside has a cleavable oxygen, a ribose ring, a removable protecting group on the cleavage oxygen and a functional group appropriate for affinity separation attached to the ribose ring.

**2.** A process according to claim 1 wherein the chemically synthesized polynucleotide has a 2'-oxygenated ribonucleotide bearing a first protecting group.

**3.** A process according to claim 2 wherein the ribonucleoside has a second protecting group on its cleavage oxygen which is different than the first protecting group on the 2'-oxygen of the ribonucleotide and which can be removed under conditions which do not remove the first protecting group from the ribonucleotide in the polynucleotide sequence.

**4.** A process according to any one of the preceding claims wherein the ribonucleoside is attached to the 5' terminus of the polynucleotide.

**5.** A process according to claim 4 wherein the polynucleotide contains a 2'-oxygenated ribonucleotide bearing the protecting group.

**6.** A process according to any one of the preceding claims wherein the process comprises a further step (b) comprising binding the polynucleotide to a binding partner appropriate for the functional group on an affinity support.

**7.** A process according to claim 6 wherein the process comprises a further step (c) comprising separating the bound functionalized polynucleotide from unlabeled failure sequences in order to yield purifed polynucleotide.

**8.** A process according to claim 7 wherein the process comprises a further step (d) comprising removing the ribonucleoside from the purified polynucleotide.

**9.** A process according to claim 8 step (d) comprises subjecting the purified polynucleotide to appropriate conditions for a period of time sufficient to cause the protecting group on the cleavage oxygen to be removed, and then subjecting the polynucleotide to basic conditions for a time sufficient to cause cleavage of the phosphodiester bond which links the ribonucleoside to the polydeoxyribonucleotide.

**10.** A process according to any one of the preceding claims wherein the ribonucleoside is attached as a phosphoramidite in the D + 1 step of the phosphoramidite chemical synthesis of a polynucleotide which contains D nucleotides.

**11.** A process according to any one of the preceding claims wherein step (a) comprises adding from about 1 to about 6 ribonucleosides in series, at least one of said ribonucleosides bearing said functional group.

**12.** A process according to any one of the preceding claims wherein the ribonucleoside is uridine.

**13.** A process according to any one of the preceding claims wherein the functional group is biotin or 2-(4-hydroxyphenylazo)benzoic acid.

**14.** A process according to any one of the preceding claims wherein the affinity support is monomeric avidin or monomeric streptavidin, preferably tetrameric avidin or tetrameric streptavidin, or an antisense polynucleotide.

**15.** A compound having the structure:

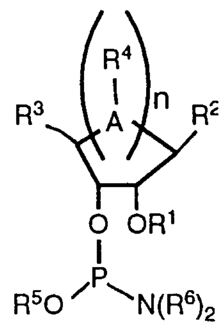

wherein **A** is oxygen, carbon, nitrogen, sulfur, or dimethylene and n is 0 or 1; $\mathbf{R^1}$ is trimethylsilyl, t-butyldimethylsilyl (t-BuMe$_2$Si), diphenylmethylsilyl, or 2'-O-[1-(2-fluorophenyl)-4-methoxypiperi-din-4-yl], O-allyl, or O-methyl, wherein t-butyldimethylsilyl (t-BuMe$_2$Si) is preferred; $\mathbf{R^2}$ and $\mathbf{R^3}$ may be the same or different and are selected from the group consisting of a functional group directly attached to the ring structure, a functional group attached to the ring structure through a spacer chain, a heterocylic base attached to the ring structure with a functional group attached to the heterocyclic base, and a heterocyclic base, a functional group, and a spacer chain, wherein the heterocyclic base is attached to the ring structure and the functional group is attached to the heterocyclic base by the spacer chain, with the proviso that only one of $\mathbf{R^2}$ and $\mathbf{R^3}$ may contain a heterocyclic base.

**16.** A compound according to claim 15 wherein the heterocyclic base present in $\mathbf{R^2}$ or $\mathbf{R^3}$ is selected from the group consisting of uracil, adenine, guanine, thymine, cytosine, and their derivatives.

**17.** A compound according to claim 15 or claim 16 wherein the spacer chain is present and is selected from the group consisting of $-(CH_2)_6$-NH, $(CH_2)_n$-**W**-, and - $[(CH_2)_y$-**V**-$(CH_2)_z]_p$-**W**-, wherein **W** is an atom which bears the functional group, and is selected from the group consisting of oxygen, nitrogen, sulfur, and an oxidized form of sulfur and **V** is selected from the group consisting of oxygen, sulfur, 1,2-phenyl, 1,3-phenyl, 1,4-phenyl, and N**Q** wherein **Q** is hydrogen or a lower alkyl containing 1 to 4 carbons.

**18.** A compound according to any one of claims 15 to 17 wherein in place of the functional group there is a reactive group suitable for subsequent attachment of a functional group is present.

**19.** A compound having the structure of either

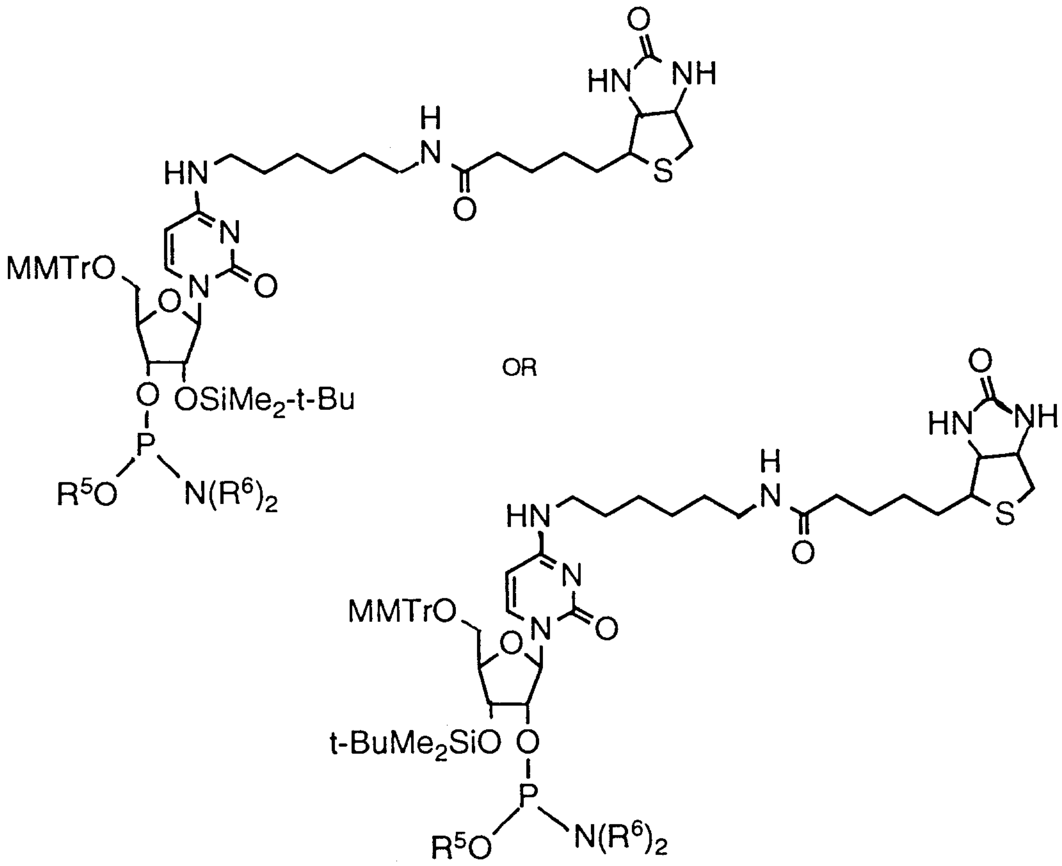

OR

wherein **R**$^5$ is beta-cyanoethyl, **R**$^6$ is isopropyl, MMTr represents methoxytrityloxymethyl and t-$BuMe_2Si$ represents t-butyldimethylsilyl.

European Patent Office

# EUROPEAN SEARCH REPORT

Application Number

EP 93 30 3123

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 067 597 (ENS BIOLOGICALS INC.) 22 December 1982 * example 2 * | 1,6-8 | C07H21/00 C07H19/10 C07H23/00 |
| D,A | NUCLEIC ACIDS RESEARCH vol. 17, no. 11, 11 October 1989, ARLINGTON, VIRGINIA US pages 7643 - 7651 ROGET A. ET AL 'Synthesis and use of labelled nucleoside phosphoramidite building blocks bearing a reporter group: biotinyl, dinitrophenyl, pyrenyl and dansyl' * page 7645 * | 1,15-19 | |
| A | EP-A-0 423 839 (CHIRON CORPORATION) 24 April 1991 * claims * | 15-19 | |
| A | DE-A-3 824 110 (MAX-PLANCK-GESELLSCHAFT ZUR FÖRDERUNG DER WISSENSCHAFT EV) 18 January 1990 * page 3 * | 1 | TECHNICAL FIELDS SEARCHED (Int. Cl.5 ) |
| | | | C07H |

The present search report has been drawn up for all claims

| Place of search | Date of completion of the search | Examiner |
|---|---|---|
| THE HAGUE | 27 AUGUST 1993 | DAY G.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document